# EUROPÄISCHE PATENTANMELDUNG

(11) **EP 1 972 623 A1**
(43) Veröffentlichungstag der Anmeldung: **24.09.2008**
(21) Anmeldenummer: 07006043.9
(22) Anmeldetag: 23.03.2007
(51) Int. Cl.: C07D 403/04, C07D 405/04, C07D 409/04, C07D 409/14, C07D 417/04, A01N 43/58

(54) **Arylpyridazine als Fungizide**

(71) Anmelder: Bayer CropScience AG, 40789 Monheim (DE)
(72) Erfinder: Gaertzen, Oliver, Dr., 50668 Köln (DE); Gayer, Herbert, Dr., 40789 Monheim (DE); Herrmann, Stefan, Dr., 40764 Langenfeld (DE); Wachendorff-Neumann, Ulrike, Dr., 56566 Neuwied (DE); Dahmen, Peter, Dr., 41470 Neuss (DE); Voerste, Arnd, Dr., 50674 Köln (DE)

(57) **Zusammenfassung**

Arylpyridazine der Formel (I) 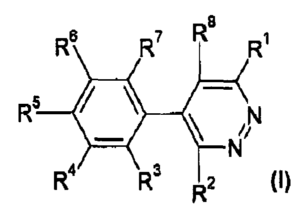 in welcher R¹, R², R³, R⁴, R⁵, R⁶, R⁷ , R⁸ und R⁹ die in der Beschreibung angegebenen Bedeutungen haben, sowie agrochemisch wirksamen Salze davon und deren Verwendung zur Bekämpfung von unerwünschten Mikroorganismen sowie Verfahren zu deren Herstellung.

Verbindungen der Formel (VII) 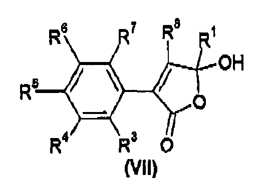 in welcher R¹, R², R³, R⁴, R⁵, R⁶, R⁷ , R⁸ und R⁹ die in der Beschreibung angegebenen Bedeutungen haben.

Verbindungen der Formel (VIII) 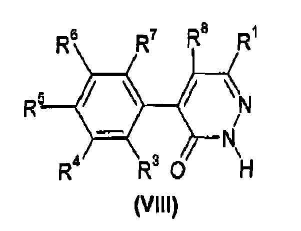 in welcher R¹, R², R³, R⁴, R⁵, R⁶, R⁷, R⁸ und R⁹ die in der Beschreibung angegebenen Bedeutungen haben.

## Beschreibung

Die Erfindung betrifft Arylpyridazine und deren Verwendung zur Bekämpfung von unerwünschten Mikroorganismen.

Es ist bereits bekannt, dass bestimmte Arylpyridazine als fungizide Pflanzenschutzmittel benutzt werden können (siehe WO-A 05/121104 und WO-A 06/001175.), Die fungizide Wirksamkeit dieser Verbindungen ist jedoch gerade bei niedrigeren Aufwandmengen nicht immer ausreichend.

Da sich die ökologischen und ökonomischen Anforderungen an moderne Fungizide laufend erhöhen, beispielsweise was Wirkspektrum, Toxizität, Selektivität, Aufwandmenge, Rückstandsbildung und günstige Herstellbarkeit angeht, und außerdem z.B. Probleme mit Resistenzen auftreten können, besteht die ständige Aufgabe, neue Fungizide zu entwickeln, die zumindest in Teilbereichen Vorteile gegenüber den bekannten aufweisen.

Überraschenderweise wurde nun gefunden, dass die vorliegenden Arylpyridazine die genannten Aufgaben zumindest in Teilaspekten lösen und sich daher als Fungizide eignen.

Gegenstand der Erfindung sind Verbindungen der Formel (I), 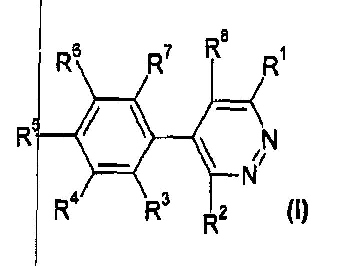 in welcher die Symbole folgende Bedeutungen haben:
- R¹: steht für C₁-C₈ alkyl, C₂-C₈-Alkonyl, C₂-C₈.Alkinyl, C₃-C₈-Cycloalkyl, C₃-C₈-Cycloalkenyl oder für einen drei- bis zehngliedrigen gesättigten, mono- oder bicyclischen C-gebundenen Heterocyclus, enthaltend ein bis vier Heteroatome aus der Gruppe O, N oder S, mit der Maßgabe dass zwei Sauerstoffatome nicht benachbart sein dürfen, und wobei R¹ durch eine bis drei gleiche oder verschiedene Gruppen R^{a} substituiert sein kann und
- R^{a}: steht für Halogen, Alkylamino, Dialkylamino, Alkylhydrazino, Cyano, Oxo, Nitro, C₁-C₆-Alkylthie, C₁-C₆-Alkyl, C₁-C₆-Halogenalkyl, tri(C₁-C₄-Alkyl)silyl) und/oder C₁-C₆-Alkoxy und/oder für unsubstituiertes, durch Halogen, C₁-C₄-Alkyl oder C₁-C₄-Halogenalkyl substituiertes C₃-C₆-Cycloalkyl,
- R²: steht für Halogen, Cyano, jeweils geradkettiges oder verzweigtes C₁-C₆-Alkoxy, C₁-C₆-Alkylthio, C₁-C₆-Alkylsulsulfinyl, C₁-C₆-Alkylsulfonyl, C₁-C₆-Halogena1koxy oder C₁-C₆-Halogenalkylthio,
- R³ bis R⁷: stehen unabhängig voneinander gleichartig oder verschieden für
Wasserstoff, Halogen, Cyano, Nitro, Amino, Hydroxy, Mercapto, Formyl, Carboxy, Carbamoyl, Thiocarbamoyl;
jeweils geradkettiges oder verzweigtes Alkyl, Alkoxy, Alkylthio, Alkylsulfinyl oder Alkylsulfonyl mit 1 bis 6 Kohlenstoffatomen;
jeweils geradkettiges oder verzweigtes Alkenyl oder Alkenyloxy mit jeweils 2 bis 6 Kohlenstoffatomen;
jeweils geradkettiges oder verzweigtes Alkinyl oder Alkinyloxy mit jeweils 2 bis 6 Kohlenstoffatomen;
jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen;
jeweils geradkettiges oder verzweigtes Halogenalkenyl oder Halogenalkenyloxy mit jeweils 2 bis 6 Kohlenstoffatomen und 1 bis 11 gleichen oder verschiedenen Halogenatomen oder geradkettiges oder verzweigtes Halogenalkinyloxy mit 2 bis 6 Kohlenstoffatomen;
jeweils geradkettiges oder verzweigtes Alkylamino, Dialkylamino, Alkylcarbonyl, Alkylcarbonyloxy, Alkoxycarbonyl, Alkylsulfonyloxy, Hydroximinoalkyl oder Alkoximinoalkyl mit jeweils 1 bis 6 Kohlenstoffatomen in den einzelnen Alkylteilen;
jeweils geradkettiges oder verzweigtes Alkoxyalkyl, Halogenalkoxyalkyl, Alkylaminoalkyl, Dialkylaminoalkyl mit jeweils 1 bis 6 Kohlenstoffatomen in den einzelnen Alkylteilen;
jeweils geradkettiges oder verzweigtes Alkoxyalkoxy, Alkoxyalkoxyalkoxy, Halogenalkoxyalkoxy, Alkylaminoalkoxy, Dialkylaminoalkoxy mit jeweils 1 bis 6 Kohlenstoffatomen in den einzelnen Alkylteilen;
Cycloalkyl mit 3 bis 8 Kohlenstoffatomen;
oder zwei benachbarte Reste R³ bis R⁷ bilden zusammen einen fünf- bis siebengliedrigen gesättigten, teilweise gesättigten oder ungesättigten substituierten oder unsubstituierten Ring ggf, enthalten bis zu zwei Heteroatome ausgewählt aus Stickstoff, Sauerstoff oder Schwefel, mit der Maßgabe dass zwei Sauerstoffatome nicht benachbart sein dürfen;
- R⁸: steht für ganz oder teilweise ungesättigtes Heterocyclyl mit fünf bis sieben Ringgliedern und ein bis vier Heteroatomen aus der Gruppe Stickstoff, Sauerstoff und Schwefel, mit der Maßgabe dass zwei Sauerstoffatome nicht benachbart sein dürfen, gegebenenfalls annelliert mit einem weiteren fünf-bis sechsgliedrigen gesättigten oder ganz oder teilweise ungesättigten carbocyclischen oder heterocyclischen Ring, wobei der heterocyclische Ring ggf. ein bis zwei zusätzliche Heteroatome aus der Gruppe Stickstoff, Sauerstoff und Schwefel enthalten kann, mit der Maßgabe dass zwei Sauerstoffatome nicht benachbart sein dürfen, wobei R⁸ einfach oder mehrfach gleichartig oder verschieden durch R⁹ substituiert ist, und
- R⁹: steht für Wasserstoff, Halogen, Cyano, Amino, Hydroxy, Mercapto, Formyl, Carboxy, Carbamoyl, Thiocarbamoyl;
jeweils geradkettiges oder verzweigtes Alkyl, Alkoxy oder Alkylthio mit jeweils 1 bis 6 Kohlenstoffatomen;
jeweils geradkettiges oder verzweigtes Halogenalkyl, Halogenalkoxy oder Halogenalkylthio mit jeweils 1 bis 6 Kohlenstoffatomen und 1 bis 13 gleichen oder verschiedenen Halogenatomen;
jeweils geradkettiges oder verzweigtes Alkylamino, Dialkylamino, Alkylcarbonyl, Alkylcarbonyloxy, Alkoxycarbonyl, Hydroximinoalkyl oder Alkoximinoalkyl mit jeweils 1 bis 6 Kohlenstoffatomen in den einzelnen Alkylteilen;
jeweils geradkettige oder verzweigtes Alkoxyalkyl, Halogenalkoxyalkyl, Alkylaminoalkyl, Dialkylaminoalkyl mit jeweils 1 bis 6 Kohlenstoffatomen in den einzelnen Alkylteilen;
jeweils geradkettiges oder verzweigtes Alkoxyalkoxy, Alkoxyalkoxyalkoxy, Halogenalkoxyalkoxy, Alkylaminoalkoxy, Dialkylaminoalkoxy mit jeweils 1 bis 6 Kohlenstoffatomen den einzelnen Alkylteilen;
unsubstituiertes oder einfach bis fünffach substituiertes Cycloalkyl mit 3 bis 8 Kohlenstoffatomen, wobei die Substituenten ausgewählt aus Fluor, Chlor, Methyl oder Trifluormethyl unabhängig voneinander gleich oder verschieden sein können;
oder zwei Rest R⁹ bilden mit dem Kohlenstoffatom, an das sie gebundenen sind, eine Carbonyl- oder Thiocarbonylgruppe,
sowie agrochemisch wirksame Salze davon.

Verbindungen der Formel (I) eignen sich sehr gut zur Bekämpfung von unerwünschten Mikroorganismen. Sie zeigen vor allem eine starke fungizide Wirksamkeit und lassen sich sowohl im Pflanzenschutz als auch im Materialschutz verwenden.

Die Verbindungen er Formel (I) können sowohl in reiner Form als auch als Mischungen verschiedener möglischer isomerer Formen, insbesondere von Stereoisomeren, wie E- und Z-, threo- und erythro-, sowie optischen Isomeren, wie R- und S-Isomeren oder Atropisomeren, gegebenenfalls aber auch von Tautomeren vorliegen. Es werden sowohl die E- als auch die Z-Isomeren, wie auch die threo- und erythro-, sowie die optischen Isomeren, beliebige Mischungen dieser Isomeren, sowie die möglichen tautomeren Formen beansprucht.

Die erfindungsgemäß Verbindungen sind durch die Formel (I) allgemein definiert,

Bevorzugt sind dabei Verbindungen der Formel (I), in welcher die Symbole folgende Bedeutung haben:
- R¹: steht für Wasserstoff, Methyl, Ethyl, n-Propyl, Isopropyl, Cyclopropyl, n-Butyl, Isobutyl, sek-Butyl, tert-Butyl, Methoxymethyl, Chlordifluormethyl, Dichlorfluormethyl, Chlorfluormethyl, Chlormethyl, Dichlormethyl, Trichlormethyl, Fluormethyl, Difluormethyl, Trifluormethyl, 2.2.2-Trifluorethyl;
- R²: steht für Fluor, Chlor, Brom, Cyano, Methoxy, Ethoxy, n-Propoxy, Isopropoxy, Methylthio, Ethylthio, n-Propylthio, Isopropylthio, Methylsulfinyl, Ethylsulfinyl, Methylsulfonyl, Ethylsulfonyl, Fluormethoxy, Difluormethoxy, Trifluormethoxy, Trifluormethylthio, 2.2.2-Trifluorethoxy;
- R³ bis R⁷: stehen unabhängig voneinander gleichartig oder verschieden für
Wasserstoff, Fluor Chlor, Brom, Jod, Cyano, Nitro, Hydroxy, Mercapto, Amino, Formyl, Methyl, Ethyl, n-Propyl, Isopropyl, n-Butyl, Isobutyl, sek-l3utyl, tert-Butyl, Chlordifluormethyl,Dichlorfluormethyl, Chlorfluormethyl, Chlormethyl, Dichlormethyl, Trichlormethyl, Fluormethyl,Difluormethyl, Trifluormethyl, 2.2-Dichlor-2-fluoroethyl, 2-Chlor-2,2-difluothyl, 2-Chlor-2-fluorethyl, 2,2.2-Trichlorethyl, 2-Fluorethyl, 2.2-Difluorethyl, 2.2.Trifluorethyl, Vinyl, Ethinyl, Allyl, Propargyl, Methoxy, Ethoxy, n-oder i-Propoxy, Methythio, Ethylthio, n-Propylthio, Isopropylthio, Methylsulfinyl, Ethylsulfinyl, Methylsulfonyl, Ethylsulfonyl, Allyloxy, Propargyloxy, Difluormethoxy, Trifluormethoxy, Difluorchlormethoxy, 2.2.2-Trifluorethoxy, Difl,uormethylthio, Difluorchlormethylthio, Trifluormethylthio, Trifluormethylsulfinyl, Trifluormethylsulfonyl, Trichlorethinyloxy, Trifluorethinyloxy, Chlorallyloxy, Methylamino, Ethylamino, n-Propylamino, Isopropylamino, Cyclopropylamino, Dimethylamino, Diethylamino, Acetyl, Propionyl, Acetyloxy, Methoxycarbonyl, Ethoxycarbonyl, Methoxymethyl, Ethoxymethyl, 2-Methoxyethoxy, 2-Ethoxyethoxy, (2-Methoxyethoxy)ethoxy, 2-(Dimethylamino)ethoxy, 2-Morpholin-4-ylethoxy, 3-(Dimethylamino)propoxy, 3-Morpholin-4-ylpropoxy, Hydroximinomethyl, Hydroximinoethyl, Methoximinomethyl, Ethoximinomethyl, N-Methoxyethanimidoyl, N-Ethoxyethanimidoyl. Cyclopropyl, Cyclobutyl, Cyclopentyl, Cyclohexyl;
oder zwei benachbarte Reste R³ bis R⁷ bilden zusammen eine 1,3-Propandiyl-, 1,4-Butandiyl-, Methylenbis(oxy)- oder Ethan-1,2-diylbis(oxy)-Gruppe, wobei diese Reste einfach oder mehrfach, gleichartig oder verschieden substituiert sein können durch Fluor, Chlor, Methyl, Ethyl , n-Propyl, Isopropyl und/oder Trifluormethyl;
wobei bis zu vier Reste R³ bis R⁷ ungleich Wasserstoff sein können;
- R⁸: steht bevorzugt für einen der nachstehend genannten heterocylischen Reste, welcher jeweils unsubstituiert oder einfach oder mehrfach durch gleiche oder verschiedenene Reste R⁹ substituiert sein kann:
2-Furyl, 3-Furyl, 2-Thienyl, 3-Thienyl, 1-Pyrrolyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 1-Pyrazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 1-Imidazolyl, 2-Imidazolyl, 4-Imidazolyl, 5-Imidazolyl, 1,2,4-Oxadiazol-3-yl, 1,2,4-Oxadiazol-5-yl, 1,2,4-Thiadiuol-3-yl, 1,2,4-Thiadiazol-5-yl, 1,3,4-Oxadiazol-2-yl, 1,3 4-Thiadiazol-2-yl, 1,2,4-Triazol-1-yl, 1,2,4-Triazvl-3-yl, 1,2,4-Triazol-4-yl, 1,2,4-Triazol-5 -yl, 1,2,3-Triazol-1-yl, 1,2,3-Triazol-2-yl, 1,2,3-Triazal-4-yl, Tetrazol-1-yl, Tctrazol-2-yl, Tetrazol-5-yl, Indol-1-yl, Indol-2-yl, Indol-3-yl, Isoindol-1-yl, Isoindol-2-yl, Benzofur-2-yl Benzothiophen-2-yl, Benzofur-3-yl, Brnzothiophen-3-yl, Benzoxazol. 2-yl, Benzothiazol2-yl, Benzimidazol-2-yl, Indazol-1-yl, Indazol-2-yl, Indazol-3-yl, 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl, 1,2,4-Triazin-3-yl, 1,2,4-Triazin-5-yl, 1,2,4-Triazin-6-yl;
- R⁹: ist gleich oder verschieden Wasserstoff, Fluor, Chlor, Brom, Iod, Cyano, Hydroxy, Mercapto, Amino, Methyl, Ethyl, n-Propyl, Isopropyl, n-Butyl, Isobutyl, sek-Butyl, tert-Butyl, Cyclopropyl, 1-Chlomyclopropyl, Methoxy, Ethoxy, Isopropoxy, Methylthio, Ethylthio, Trifluormethylthio, Chlordifluormethyl, Dichlorfluormethyl, Chlorfluormethyl,
Chlormethyl, Dichlormethyl, Trichlormethyl, Fluormethyl, Difluormethyl, Trifluormethyl, 2,2.2-Trifluorethyl, Trifluormethoxy, Trifluormethylthio, 2.2.2-Trifluorethoxy, 2.2-Dichlor-2-fluorethyl 2,2-Difluor-2-chlorethyl, 2-Chlor-2-fluorethyl, 2,2,2-Trichlorethyl, 2.2.2-Trifluorethyl, 2-Fluorethyl, 2,2-Difluorethyl, 2-Methoxyethoxy, Acetyl, Propionyl, Methoxycarbonyl, Ethoxycarbonyl, N-Methylamino, N,N-Dimethylamino, N-Ethylamino, N,N'-Diethylamino;
oder zwei Reste R⁹ bilden mit dem Kohlenstoffatom, an das sie gebundenen sind, eine Carbonyl- oder Thiocarbonylgruppe,
sowie agrochemisch wirksame Salze davon.

Besonders bevorzugt sind dabei Verbindungen der Formel (I), in welchen die Symbole folgende Bedeutung haben:
- R¹: steht für Methyl, Ethyl, Cyclopropyl, Fluormethyl, Difluormethyl, Trifluormethyl;
- R²: steht für Fluor, Chlor, Brom, Cyano;
- R³: steht für Fluor, Chlor, Brom, Cyano, Methyl, Difluormethyl, Trifluormethyl, Trifluormethoxy, Methoxy;
- R⁴ bis R⁷: stehen unabhängig voneinander gleichartig oder verschieden für:
Wasserstoff, Fluor, Chlor, Brom, Iod, Cyano, Methyl, Ethyl, n-Propyl, Isopropyl, Isobutyl, tert-Butyl, Cyclopropyl Methoxy, Ethoxy, Methylthio, Difluormethyl, Trifluormethyl, 2.2.2-Trifluorethyl, Trifluormethoxy, Trifluormethylthio, 2-Methoxyethoxy, (2-Methoxyethoxy)ethoxy, 2-(Dimethylamino)ethoxy, 2-Morpholin-4-ylethoxy, 3-(Dimethylamino)propoxy, 3-Morpholin-4-ylpropoxy;
oder zwei benachbarte Reste R³ bis R⁷ bilden zusammen eine Methylenbis(oxy)- oder Ethan. 1,2-diylbis(oxy)-Gruppe, wobei diese Reste einfach oder mehrfach, gleichartig oder verschieden durch Fluor oder Methyl substituiert sein können;
wobei bis zu drei Reste R⁴ bis R⁷ ungleich Wasserstoff sein können;
- R⁸: steht für einen der nachstehend genannten heterocylischen Reste, welcher jeweils unsubstituiert oder einfach oder mehrfach durch gleiche oder verschiedenene Reste R⁹ substituiert sein kann: 2-Furyl, 3-Furyl, 2-Thienyl, 3-Thienyl, 1-Pyrrolyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 1-Pyrazolyl, 3-Pyrazolyl, 4-Pyrazo 5-Pyrazolyl, 2-Oxazolyl, 4-Oxazolyl, 5-Oxazolyl, 2-Thiazolyl, 4-Thiazolyl, 5-Thiazolyl, 1-Imidazolyl, 2-Imidazolyl, 4-Imidazolyl, 5-Imidazolyl, 1,2,4-Oxadiazol-3-yl, 1,2,4-Oxadiazol-5-yl, 1,3,4-Oxadiazol-2-yl, 1,2,4-Thiadiazol-3-yl, 1,2,4-Thiadiazol-5-yl, 1,3,4-Thiadiazol-2-yl, 1,2,4-Triazol-1-yl, 1,2,4-Triazol-3-yl, 1,2,4-Triazol. 4-yl, 1,2,4-Triazol-5-yl, Tetrazol-5-yl, 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl, 1,2,4-Triazin-3-yl, 1,2,4-Triazin-5-yl, 1,2,4-Triazin-6-yl;
- R⁹: ist gleich oder verschieden Wasserstoff, Fluor, Chlor, Brom, Iod, Cyano, Hydroxy, Mercapto, Methyl, Ethyl, n-Propyl, Isopropyl, n-Butyl, Isobutyl, sek-Butyl, tert.-Butyl, Cyclopropyl, Methoxy, Ethoxy, Methylthio, Ethylthio, Trifluormethylthio, Chlordifluormethyl, Dichlorfluormethyl, Chlorfluormethyl, Chlormethyl, Dichlormethyl, Trichlormethyl, Difluormethyl, Trifluormethyl , 2,2.2-Trifluorethyl, Trifluormethoxy, 2.2.2-Trifluorethox , 2-Methoxyethoxy, Acetyl, Propionyl, Methoxycarbonyl, Ethoxycarbonyl, N,N-Dimethylamino;
oder zwei Reste R⁹ bilden mit dem Kohlenstoffatom, an das sie gebundenen sind, eine Carbonyl- oder Thiocarbonylgruppe,
sowie agrochemisch wirksame Salze davon.

Ganz besonders bevorzugt sind dabei Verbindungen der Formel (I), bei denen die Reste R¹ bis R⁷ und R⁹ die oben angegebene Bedeutung haben und
- R⁸: ist gleich 2-Furyl, 3-Furyl.

Weiterhin ganz besondere bevorzugt sind Verbindungen der Formel (I), bei denen die Reste R¹ bis
- R⁷ und R⁹: die oben angegebene Bedeutung haben und
- R⁸: ist gleich 2-Thieny 3-Thienyl.

Weiterhin ganz besonders bevorzugt sind Verbindungen der Formel (I), bei denen die Reste R¹ bis
- R⁷ und R⁹: die oben angegebene Bedeutung haben und
- R⁸: ist gleich 1-Pyrrol , 2-Pyrrolyl, 3-Pyrrolyl.

Weiterhin ganz besonders bevorzugt sind Verbindungen der Formel (I), bei denen die Reste R¹ bis
- R⁷ und R⁹: die oben angegebene Bedeutung haben und
- R⁸: ist gleich 3-Isoxazolyl, 4-Isoxazolyl, 5-Isoxazolyl.

Weiterhin ganz besonders bevorzugt sind Verbindungen der Formel (I), bei denen die Reste R¹ bis
- R⁷ und R⁹: die oben angegebene Bedeutung haben und
- R⁸: ist gleich 3-Isothiazolyl, 4-Isothiazolyl, 5-Isothiazolyl.

Weiterhin ganz besonders bevorzugt sind Verbindungen der Formel (I), bei denen die Reste R¹ bis
- R⁷ und R⁹: die oben angegebene Bedeutung haben und
- R⁸: ist gleich 1-Pyrazolyl, 3-Pyrazolyl, 4-Pyrazolyl, 5-Pyrazolyl.

Weiterhin ganz besonders bevorzugt sind Verbindungen der Formel (I), bei denen die Reste R¹ bis
- R⁷ und R⁹: die oben angegebene Bedeutung haben und
- R⁸: ist gleich 2-Oxazolyl, 4-Oxazolyl, 5-Oxazolyl ,

Weiterhin ganz besonders bevorzugt sind Verbindungen der Formel (I), bei denen die Reste R¹ bis
- R⁷ und R⁹: die oben gegebene Bedeutung haben und
- R⁸: ist gleich 2-Thiazolyl, 4-Thiazolyl, 5-Thiazolyl.

Weiterhin ganz besonders bevorzugt sind Verbindungen der Formel (I), bei denen die Reste R¹ bis
- R⁷ und R⁹: die oben angegebene Bedeutung haben und
- R⁸: ist gleich 1-Imidazolyl,2-Imidazolyl, 4-Imidazolyl, 5.Imidazolyl.

Weiterhin ganz besonders bevorzugt sind Verbindungen der Formel (I), bei denen die Reste R¹ bis
- R⁷ und R⁹: die oben gegebene Bedeutung haben und
- R⁸: ist gleich 1,2,4-Oxadiazol-3-yl, 1,2,4-Oxadiazol-5-yl, 1,3,4-Oxadiazol-2-yl.

Weiterhin ganz besonders bevorzugt sind Verbindungen der Formel (I), bei denen die Reste R¹ bis
- R⁷ und R⁹: die oben gegebene Bedeutung haben und
- R⁸: ist gleich 1,2,4-Thidiazol-3-yl, 1,2,4-Thiadiazol-5-yl, 1,3,4-Thiadiazol-2-yl,

Weiterhin ganz besonders bevorzugt sind Verbindungen der Formel (I), bei denen die Reste R¹ bis
- R⁷ und R⁹: die oben angegebene Bedeutung haben und
- R⁸: ist gleich 1,2,4-Triazol-1-yl, 1,2,4-Triazol-3-yl, 1,2,4-Triazol-4.y4 1,2,4-Triazol-5-yl.

Weiterhin ganz besonders bevorzugt sind Verbindungen der Formel (I), bei denen die Reste R¹ bis
- R⁷ und R⁹: die oben angegebene Bedeutung haben und
- R⁸: ist gleich Tetrazol-5-yl.

Weiterhin ganz besonders bevorzugt sind Verbindungen der Formel (I), bei denen die Reste R¹ bis
- R⁷ und R⁹: die oben angegebene Bedeutung haben und
- R⁸: ist gleich 2-Pyridiny , 3-Pyridinyl, 4-Pyridinyl.

Weiterhin ganz besonders bevorzugt sind Verbindungen der Formel (I), bei denen die Reste R¹ bis
- R⁷ und R⁹: die oben angegebene Bedeutung haben und
- R⁸: ist gleich 3-Pyridazinyl, 4-Pyridazinyl.

Weiterhin ganz besonders bevorzugt sind Verbindungen der Formel (I), bei denen die Reste R¹ bis
- R⁷ und R⁹: die oben angegebene Bedeutung haben und
- R⁸: ist gleich 2-Pyrimid 1, 4-Pyrimidinyl, 5-Pyrimidinyl.

Weiterhin ganz besonders bevorzugt sind Verbindungen der Formel (I), bei denen die Reste R¹ bis
- R⁷ und R⁹: die oben gegebene Bedeutung haben und
- R⁸: ist gleich 2-Pyrazinyl.

Weiterhin ganz besonders bevorzugt sind Verbindungen der Formel (I), bei denen die Reste R¹ bis
- R⁷ und R⁹: die oben angegebene Bedeutung haben und
- R⁸: ist gleich 1,3,5-Triazin-2-yl.

Weiterhin ganz besonders bevorzugt sind Verbindungen der Formel (I), bei denen die Reste R¹ bis
- R⁷ und R⁹: die oben angegebene Bedeutung haben und
- R⁸: ist gleich 1,2,4-Triazin-3-yl, 1,2,4-Triazin-5-yl,1,2,4-Triazin-6-yl.

Insbesondere bevorzugt sind Verbindungen der Formel (I), bei denen die Reste R⁸ und R⁹ die oben angegebenen Bedeutungen haben und
- R¹: steht für Methyl, Ethyl;
- R²: steht für Fluor, Chlor, Brom;
- R³: steht für Wasserstoff, Fluor, Chlor, Brom, Methyl, Methoxy;
- R⁴ bis R⁷: stehen unabhängig voneinander gleichartig oder verschieden für:
Wasserstoff, Fluor, Chlor, Cyano, Methyl;
oder zwei benachbarte Reste R³ bis R⁷ bilden zusammen eine Methylenbis(oxy) oder Ethan-1,2-diylbis(oxy)-Gruppe, wobei diese Reste einfach oder mehrfach, gleichartig oder verschieden durch Fluor oder Methyl substituiert sein können;
wobei bis zu drei Reste R⁴ bis R⁷ ungleich Wasserstoff sein können;
sowie agrochemisch wirksame Salze davon.

Die zuvor genannten Reste-Definitionen können untereinander in beliebiger Weise kombiniert werden, Außerdem können einzelne Definitionen entfallen.

Je nach Art der oben definierten Substituenten weisen die Verbindungen der Formel (I) saure oder basische Eigenschaften auf und können mit anorganischen oder organischen Säuren oder mit Basen oder mit Metallionen Salze, gegebenenfalls auch innere Salze oder Addukte bilden, Tragen die Verbindungen der Formel (I) Amino, Alkylamino oder andere, basische Eigenschaften induzierende Gruppen, so können diese Verbindungen mit Säuren zu Salzen umgesetzt werden oder fallen durch die Synthese direkt als Salze an,

Beispiele für anorganische Säuren sind Halogenwasserstoffsäuren wie Fluorwasserstoff, Chlorwasserstoff, Bromwasserstoff und Iodwasserstoff, Schwefelsäure, Phosphorsäure und

Salpetersäure und saure saite wie NaHSO₄ und KHSO₄.

Als organische, Säuren kommen beispielsweise Ameisensäure, Kohlensäure und Alkansäuren wie Essigsäure, Trifluoressigsäure, Trichloressigsäure und Propionsäure sowie Glycolsäure, Thiocyansäure, Milchsäure Bernsteinsäure, Zitronensäure, Benzoesäure, Zimtsäure, Oxal-säure, Alkylsulfonsäuren (Sulfonsäuren mit geradkettigen oder verzweigten Alkylresten mit 1 bis 20 Kohlenstoffatomen), Arylsulfonsäuren oder -disulfonsäuren (aromatische Reste wie Phenyl und Naphthyl welche ein oder zwei Sulfonsäuregruppen tragen), Alkylphosphon-säuren (Phosphonsäuren mit geradkettigen oder verzweigten Alkylresten mit 1 bis 20 Kohlenstoffatomen), Arylphosphonsäuren oder - diphosphonsäuren (aromatische Reste wie Phenyl und Naphthyl welche ein oder zwei Phosphonsäurereste tragen), wobei die Alkyl- bzw, Arylreste weitere Substituenten tragen können, z.B, p-Toluo1sulfonsiture, Salicylsäure, p-Aminosalicylsäure, 2-Phenoxybenzoesäure, 2-Acetoxybenzoesäure etc.

Die so erhältlichen Salze weisen ebenfalls fungizide Eigenschaften auf,
Bei den in den vorstehenden Formeln angegebenen Definitionen der Symbole wurden Sammelbegriffe verwendet, die allgemein repräsentativ für die folgenden Substituenten stehen:
**Halogen:** Fluor, Chlor, Brom und Jod;
**Alkyl:** gesättigte, geradkettige oder verzweigte Kohlenwasserstoffreste mit 1 bis 8 Kohlenstoffatomen, z.B. C₁-C₆-Alkyl wie Methyl, Ethyl, Propyl, 1-Methylothyl, Butyl, 1-Methyl-propyl, 2-Methylpropyl, 1,1-Dimeth/lethyl, Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl,1-Methylpentyl, 2-Methylpentyl, 3-Methylpeltyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl;
**Halogenalkyl:** geradkettige oder verzweigte Alkylgruppen mit 1 bis 8 Kohlenstoffatomen (wie vorstehend genannt), wobei in diesen Gruppen teilweise oder vollständig die Wasserstoffatome durch Halogenatome wie vorstehend genannt ersetzt sein können, z.B. C₁-C₂-Halogenalkyl wie Chlormethyl, Brommethyl, Dichlormethyl, Trichlormethyl, Fluormethyl, Difluormethyl, Trifluor-methyl, Chlorfluormethyl, Dichlorfluormethyl, Chlordifluormethyl, 1-Chlorethyl, 1-Bromethyl, 1-Fluorethyl, 2-Fluorothyl, 2,2-Difluorethyl, 2,2,2-Trifluorethyl, 2-Chlor-2-fluorethyl, 2-Chlor,2.di-fluorethyl, 2,2-Dichlor-2-fluorethyl, 2,2,2-Trichlorethyl, Pentafluorethyl und 1,1,1-Trifluorprop-2-yl;
**Halogenalkoxy:** geradkettige oder verzweigte Alkoxygruppen mit 1 bis 8 Kohlenstoffatomen (wie vorstehend genannt), wobei in diesen Gruppen teilweise oder vollständig die Wasserstoffatome durch Halogenatome wie vorstehend genannt ersetzt sein können, z.B. C₁-C₂-Halogenalkoxy wie Chlormethoxy, Brommethoxy , Dichlormethoxy, Trichlormethoxy, Fluormethoxy, Difluormethoxy, Trifluormethoxy, Chlorfluormethoxy, Dichlorfluormethoxy, Chlordifluormethoxy, 1-Chlorethoxy, 1-Bromethoxy, 1-Fluorethoxy,-2-Fluorethoxy, 2,2-Difluorethoxy, 2,2,2-Trifluorethoxy, 2-Chlor-2-fluorethoxy, 2-Chlor,2-difluorethoxy, 2,2-Dichlor-2-fluorethoxy, 2,2,2-Trichlorethoxy, Pentafluorethoxy und 1,1,1-Trifluorprop-2-oxy;
**Halogenalkylthio:** geradkettige oder verzweigte Alkylthiogruppen mit 1 bis 8 Kohlenstoffatomen (wie vorstehend genannt), wobei in diesen Gruppen teilweise oder vollständig die Wasserstoffatome durch Halogenatome wie vorstehend genannt ersetzt sein können, z.B. C₁-C₂-Halogenalkylthio wie Chlormethylthio, Brommethylthio, Dichlormethylthio, Trichlormethylthio, Fluormethylthio, Difluormethylthio, Trifluormethylthio, Chlorfluormethylthio, Dichlorfluormethylthio, Chlordifluormethylthio, 1-Chlorethylthio, 1-Bromethylthio, 1-Fluorethylthio, 2-Fluorethylthio, 2,2-Difluorethylthio, 2,2,2-Trifluorethylthio, 2-Chlor-2.fluorethyltbio, 2-Chlor,2-difluorethylthio, 2,2-Dichlor-2.fluorethyllhio, 2,2,2-Trichlorethylthio, Pentafluorothylthio und 1,1,1-Trifluorprop-2-ylthio-,
**Alkenyl:** ungesättigte, geradkettige oder verzweigte Kohlenwasseratoffresta mit 2 bis 8 Kohlenstoffatomen und einer Doppelbindung in einer beliebigen Position, z.B. C₂-C₆-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butorryl, 2-Methyl-l.butenyl, 3-Methyl-l-butonyl, 1-Mothyl-2-butenyl, 2-Methyl-2-butenyl, 3-Methyl-2-butenyl, 1-Methyl-3-butanyl, 2-Mothyl-3-butenyl, 3-Methyl-3-butenyl 1,1-Dimethyl-2-propenyl, 1,2-Dimethyl-1-propenyl, 1,2-Dimethyl-2-propenyl, 1-Ethyl-1-propen 1, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Mothyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Me liyl-4-pentenyl, 4-Methyl-4-pentenyl, 1,1-Dimethyl-2-butenyl, 1,1,-Dimethyl-3 -butenyl, 1,2-D methyl-1-butenyl, 1,2-Dimethyl-2-butenyl, 1,2-Dimethyl-3-butenyl, 1,3-Dimethyl-1-butenyl, 1,3 Dimethyl-2-butenyl, 1,3-Dimethyl-3-butenyl, 2,2-Dimethyl-3-butenyl, 2,3-Dimethyl-1-butenyl, 2,3 Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyll-3-butenyl, 1,1,2-Trimethyl-2-propenyl, 1-Ethyl-1-methyl-2-propenyl, 1-Ethyl-2-methyl 1-proponyl und 1-Ethyl-2-methyl-2-propenyl;
**Alkinyl:** geradkettige oder verzweigte Kohlenwasserstoffgruppen mit 2 bis 8 Kohlenstoffatomen und einer Dreifachbindung in einer beliebigen Position, z,B, C₂-C₆-Alkinyl wie Ethinyl, 1-Propinyl, 2-Propinyl, 1-Butinyl, 2.Butinyl, 3.Butinyl, 1-Mothyl-2-propinyl, 1-Pentinyl, 2-Pentinyl, 3-Pentinyl, 4-Pentinyl, 1- ethyl-2-butinyl, 1-Methyl-3-butinyl, 2-Methyl-3-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 1-Methyl-2-pentinyl, 1-Methyl-3-pentinyl, 1-Methyl-4.pentinyl, 2-Methyl-3-pentinyl, 2-Methyl-4-pentinyl, 3-Methyl-1-pentinyl, 3-Methyl-4-pentinyl, 4-Methyl-1-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl.3-butinyl, 2-Ethyl-3-butinyl und 1-Ethyl-1-methyl-2-propinyl;
**Alkoxy:** gesättigte, geradkettige oder verzweigte Alkoxyreste mit 1 bis 8 Kohlenstoffatomen, z.B. C₁-C₆-Alkoxy wie Methoxy, Ethoxy, Propoxy, 1-Methylethoxy, Butoxy, 1-Methyl-propoxy, 2-Methylpropoxy, 1,1-Dimethylethoxy, Pentoxy, 1-Methylbutoxy, 2-Methylbutoxy, 3-Methylbutoxy, 2,2-Di-methylpropoxy, 1-Ethylpropoxy, Hexoxy, 1,1-Dimethylpropoxy, 1,2-Dimethylpropoxy,1-Methylpentoxy, 2-Methylpentoxy, 3-Methylpentoxy, 4-Methylpentoxy, 1,1-Dimethylbutoxy, 1,2-Dimethylbutoxy, 1,3-Dimethylbutoxy, 2,2-Dimethylbutoxy, 2,3-Dimethylbutoxy, 3,3-Dimethylbutoxy, 1-Ethylbutoxy, 2-Ethylbutoxy, 1,1,2-Trimethylpropoxy, 1,2,2-Trimethylpropoxy, 1-Ethyl-1-methylpropoxy und 1-Ethyl-2-methylpropoxy,
**Alkylamino:** gesättigte, geradkettige oder verzweigte Alkylaminoreste mit 1 bis 8 Kohlenstoffatomen, z.B. C₁-C₆-Alkylamino wie Methylamino, Ethylamine, Propylamino, 1-Methylethylamino, Butylamino, 1-Methyl-propylamino, 2-Methylpropylamino, 1,1-Dimethylethylamino, Pentylamino, 1-Methylbutylamino, 2-Methylbutylamino, 3-Methylbutylamino, 2,2-Di-methylpropylamino, 1-Ethylpropylamino, Hexylamino, 1,1-Dimethylpropylamino, 1,2-Dimethylpropylamino, 1-Methylpentylamino, 2-Methylpentylamino, 3-Methylpentylamino, 4-Methytpentylamino, 1,1-Dimethylbutylamino, 1,2-Dimethylbutylamino, 1,3-Dimethylbutylamino, 2,2-Dimethylbutylamino, 2,3-Dimethylbutylamino, 3,3-Dimethylbutylamino, 1-Ethybutylamino, 2-Ethylbutylamino, 1,1,2-Trimethylpropylamino, 1,2,2-Trimethylpropylamino, 1-Ethyl-1-methylpropylamino und 1-Ethyl-2-methylpropylamino;
**Alkylthio:** gesättigte, geradkettige oder verzweigte Alkylthioreste mit 1 bis 8 Kohlenstoffatomen, z.B. C₁-C₆-Alkylthio wie Methylthio, Ethylthio, Propylthio, 1.Methylethylthio, Butylthio, 1-Methyl-propylthio, 2-Methylpropylthio, 1,1-Dimethylethylthio, Pentylthio, 1-Mothylbutylthio, 2. Methylbutylthio, 3-Methylbntylthio, 2,2-Di-methylpropylthio, 1-Ethylpropylthio, Hexylthio, 1,1-Dimethylpropylthio, 1,2-Dimethylpropylthio,1-Methylpentylthio, 2-Methylpentylthio, 3. Methylpentyhhio, 4-Methylpentylthio, 1,1-Dimethylbutylthio, 1,2-Dimethylbutylthio, 1,3-Dimethylbutylthio, 2,2-Dimethytbutylthio, 2,3-Dimethylbutylthio, 3,3-Dimethylbutylthio, 1-Ethylbutylthio, 2-Ethylbutylthio, 1,1,2-Trimethylpropylthio, 1,2,2-Trimethylpropylthio, 1-Ethyl-1-methylpropylthio und 1-Ethyl-2-methylpropylthio;
**Alkylsulfinyl:** gesättigte, geradkettige oder verzweigte Alkylsulfinylreste mit 1 bis 8 Kohlenstoffatomen, z.B. C₁-C₆-Alkylsulfinyl wie Methylsulfinyl, Ethylsulfinyl, Propylsulfinyl, 1-Methylethylsulfinyl, Butylsulfinyl, 1-Methyl-propylsulfinyl, 2-Methylpropylsulfinyl, 1,1-Dimethylethylsulfinyl, Pentylsulfinyl, 1-Methylbutylsulfinyl, 2-Methylbutylsulfinyl, 3-Methylbutylsulfinyl, 2,2-Di-methylpropylsulfinyl, 1-Ethylpropylsulfinyl, Hexylsulfinyl, 1,1-Dimethylpropylsulfinyl, 1,2-Dimethylpropylsulfinyl, 1-Mothylpentylsulfinyl, 2-Methylpentylsulfinyl, 3-Mithylpentylsulfinyl, 4-Methylpentylsulfinyl, 1,1-Dimethylbutylsulfinyl, 1,2-Dimethylbutylsulfinyl, 1,3-Dimethylbutylsulfinyl, 2,2-Dimethylbutylsulfinyl, 2,3-Dimethylbutylsulfinyl, 3,3-Dimethylbutylsulfinyl, 1-Ethylbutylsulfinyl, 2-Ethylbutylsulfinyl, 1,1,2-Trimethylpropylsulfinyl, 1,2,2-Trimethylpropylsulfinyl, 1-Ethyl-1-methylpropylsulfinyl und 1-Ethyl-2-methylpropylsulfinyl;
**Alkylsulfonyl:** gesättigte, geradkettige oder verzweigte Alkylsulfonylreste mit 1 bis 8 Kohlenstoffatomen, z,B, C₁-C₆-Alkylsulfonyl wie Methylsulfonyl, Ethylsulfonyl, Propylsulfonyl, 1-Methylethylsulfonyl, Butylsulfonyl, 1-Methyl-propylsulfonyl, 2-Methylpropylsulfonyl, 1,1-Dimethylethylsulfonyl, Pentylsulfonyl, 1-Methylbutylsulfonyl, 2-Methylbutylsulfonyl, 3-Methylbutylsulfonyl, 2,2-Di-methylpropylsulfonyl, 1-Ethylpropylsulfonyl, Hexylsulfonyl, 1,1-Dimethylpropylsulfonyl, 1,2-Dimethylpropylsulfonyl,1-Methylpentylsulfonyl, 2-Methylpentylsulfonyl, 3-Methylpentylsulfonyl, 4-Methylpentylsulfonyl, 1,1-Dimethylbutylsulfonyl, 1,2-Dimethybulylsulfonyl, 1,3-Dimethylbutylsulfonyl, 2,2,Dimethylbutylsulfonyl, 2,3-Dimethylbutylsulfonyl, 1,3-Dimethylbutylsulfonyl, 1-Ethylbutylsulfonyl, 2-Ethylbutylsulfonyl, 1,1,2-Trimethylpropylsulfonyl, 1, 1,2,2-Trimethytpropytsulfonyl, 1-Ethyl-1-methylpropylsulfonyl und 1-Ethyl-2-methylpropyl sulfonyl;
**Cycloalkyl:** monocyclische gesättigte Kohlenwasserstoffgruppen mit 3 bis 6 Kohlenstoffringgliedern, wie Cyclopropyl, Cyclobutyl, Cyclopentyl und Cyclohexyl;
**Cylcoalkenyl:** monocyclische, nicht aromatische Kohlenwasserstoffgruppen mit 3 bis 8 Kohlenstoffringgliedern mit mindestens einer Doppelbindung, wie Cyclopenten-1-yl, Cyclohexen-1-yl, Cyclohepta-1,3-dien-1-yl;
**Alkoxycarbonyl:** eine Alkoxygruppe mit 1 bis 6 Kohlenstoffatomen (wie vorstehend genannt), welche über eine Carbonylgruppe (-CO-) an das Gerüst gebunden ist;
**Oxyalkylenoxy:** divalente unverzweigte Ketten aus 1 bis 3 CH₂-Gruppen, wobei beide Valenzen über ein Sauerstoffatom an das Gerüst gebunden ist, z.B. OCH₂O, OCH₂CH₂O und OCH₂CH₂CH₂O;

**drei- bis zehngliedriger gesättigter oder partiell ungesättigter Heterocyclus, enthaltend ein bis vier Heteroatome aus der Gruppe Sauerstoff, Stickstoff oder Schwefel:** mono- oder bicyclische Heterocyclen (Heterocyclyl) enthaltend neben Kohlenstoffringgliedern ein bis drei Stickstoffatome und/oder ein Sauerstoff- oder Schwefelatom oder ein oder zwei Sauerstoff- und/oder Schwefelatome; enthält der Ring mehrere Sauerstoffatome, so stehen diese nicht direkt benachbart; z.B. Oxiranyl, 1, Aziridinyl, 2-Tetrahydrofuranyl, 3-Tetrahydrofuranyl, 2. Tetrahydrothienyl, 3-Tetrahydrothienyl, 2-Pyrrolidinyl, 3-Pyrrolidinyl, 3-Isoxazolidinyl, 4-Isoxazolidinyl, 5-Isoxazolidinyl, 3-Isothiazolidinyl, 4.Isothiazohdinyl, 5-Isothiazolidinyl, 3-Pyrazolidinyl, 4-Pyrazolidinyl, 5-Pyrazolidinyl, 2-Oxazolidinyl, 4-Oxazolidinyl, 5-Oxaaolidinyl, 2-Thiazolidinyl, 4-thiazolidinyl, 5-Thiazolidinyl, 2-Imidazolidinyl, 4-Imidazolidinyl, 1,2,4-Oxadiazolidin-3-yl, 1,2,4-Oxadiazolidin-5-yl, 1,2,4-Thiadiazolidin-3-yl, 1,2,4-Thiadiazolidin-5-yl, 1,2,4-Triazolidin-3-yl, 1,3,4-Oxadiazolidin-2-yl, 1,3,4-Thiadiazolidin-2-yl, 1,3,4-Triazolidin-2-yl, 2,3-Dihydrofur-2-yl, 2,3-Dihydrofur-3-yl, 2,4-Dihydrofur-2-yl, 2,4-Dihydrofur-3-yl, 2,3-Dihydrothien-2-yl, 2,3-Dihydrothien-3-yl, 2,4-Dihydrothien-2-yl, 2,4-Dihydrothien-3-yl, 2-Pyrrolin-2-yl, 2-Pyrrolin-3-yl, 3-Pyrrolin-2-yl, 3-Pyrrolin-3-yl, 2-Isoxazolin-3-yl, 3-Isoxazolin-3-yl, 4-Isoxazolin-3-yl, 2-Iso azolin-4-yl, 3-Isoxazolin-4-yl, 4.Isoxazolin-4-yl, 2-Isoxazolin-5-yl, 3-Isoxazolin-5-yl, 4-Isoxazolin-5-yl, 2-Isothiazolin-3-yl, 3-Isothiazolin-3-yl, 4-Isothiazolin-3-yl, 2-Isothiazolin-4-yl, 3-Isothiazolin-4-yl, 4-Isothiazofin-4-yl, 2-Isothiazolin-5-yl, 3-Isothiazolin-5-yl, 4-Isothiazoh-5-yl, 2,3-Dihydropyrazol-1-yl, 2,3-Dihydropyrazol-2-yl, 2,3-Dihydropyrazol-3-yl, 2,3-Dihydropyrazol-4-yl, 2,3-Dihydropyrazol-5-yl, 3,4-DihydMpyrazoM.yl, 3,4-Dibydropyrazol-3-yl, 3,4-Dihydropyrazol-4-yl, 3,4-Dihydropyrazol-5-yl, 4,5-Dihydroopyrazol-1-yl, 4,5-Dihydropyrazol-3-yl, 4,5-Dihydropyrazol-4-yl, 4,5-Dihydropyrazol-5-yl, 2,3-Dihydrooxazol-2-yl, 2,3-Dihydrooxazol-3-yl, 2,3-Dihydrooxazol-4-yl, 2,3-Dihydrooxazol-5-yl, 3,4-Dihydrooxazol-2-yl, 3,4.Dihydrooxazol-3-yl, 3,4-Dihydrooxazol-4-yl, 3,4-Dihydrooxazol-5-yl, 3,4-Dihydrooxazol-2-yl, 3,4-Dihydrooxazol-3-yl, 3,4-Dihydrooxazol-4-yl, 2-Piperidinyl, 3-Piperidinyl, 4-Piperidinyl, 1,3-Dioxan-5-yl, 2-Tetrahydropyranyl, 4-Tetrahydropyranyl, 2-Tetrahydrothienyl, 3-Hexahydropyridazinyl, 4-Hexahydropyridazinyl, 2-Hexahydropyrimidinyl, 4-Hexahydropyrimidinyl, 5-Hexahydropyrimidinyl, 2-Piperazinyl, 1,3,5-Hexahydro-triazin-2-yl und 1,2,4-Haxahydrotriazin-3-yl;

**fünf- bis zehngliedriger aromatischer Heterocyclus, enthaltend ein bis vier Heteroatome aus der Gruppe Sauerstoff, Stickstoff oder Schwefel:** ein- oder zweikerniges Heteroaryl, z,B.
- 5-gliedriges Heleroaryl, enthaltend ein bis vier Stickstoffatome oder ein bis drei Stickstoffatome und ein Schwefel- oder Sauerstoffatom: 5-Ring Heteroarylgruppen, welche neben Kohlenstoffatomen ein bis vier Stickstoffatome oder ein bis drei Stickstoffatome und ein Schwefel- oder Sauerstoffatom als Ringglieder enthalten können, z.B. 2-Furyl, 3-Furyl, 2-Thienyl, 3-Thienyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, 1,2,4-Oxadiazol-3-yl, 1,2,4-Oxadiazol-5-yl, 1,2,4-Thiad azol-3-yl, 1,2,4-Thiadiazol-5-yl, 1,2,4-Triazol-3-yl, 1,3,4-Oxadiazol-2-yl, 1,3,4-Thiadiazol-2-yl und 1,3,4-Triazol-2-yl;
- **benzokondensiertes 5-gliedriges Heteroaryl, enthaltend ein bis drei Stickstoffatome oder ein Stickstoffatom und ein Sauerstoff- oder Schwefelatom:** 5-Ring Heteroarylgruppen, welche neben Kohlenstoffatomen ein bis vier Stickstoffatome oder ein bis drei Stickstoffatome und ein Schwefel- oder Sauerstoffatom als Ringglieder enthalten können, und in welchen zwei benachbarte Kohlenstoffringglieder oder ein Stickstoff- und ein benachbartes Kohlenstoffringglied durch eine Buta-1,3-dien-1,4-diylgruppe verbrückt sein können, in der ein oder zwei C-Atome durch N-Atome ersetzt sein können;
- **über Stickstoff gebundenes 5-gliedriges Heteroaryl, enthaltend ein bis vier Stickstoffatome, oder über Stickstoff gebundenes benzokondensiertes 5-gliederiges Heteroaryl, enthaltend ein bis drei Stickstoffatome:** 5-Ring Heteroarylgruppen, welche neben Kohlenstoffatomen ein bis vier Stickstoffatome bzw, ein bis drei Stickstoffatome als Ringglieder enthalten können, und in welchen zwei benachbarte Kohlensioffringglieder oder ein Stickstoff- und ein benachbartes Kohlenstoffringglied durch eine Buta-1,3-dien-1,4-diylgruppe verbrückt sein können, in der ein oder zwei C-Atome durch N-Atome ersetzt sein können, in der ein oder zwei C-Atome durch N.Atome ersetzt sein können, wobei diese Ringe über eines der Stickstoffringglieder an das Gerüst gebunden sind, z.B. 1-Pyrrolyl, 1-Pyraxolyl, 1,2,4-Triazol-1-yl, 1-Imidazolyl, 1,2,3-Triazol-1-yl, 1,3,4-Triazo1-1-yl;-

**6-gliedriges Heteroaryl, enthaltend ein bis drei bzw, ein bis vier Stickstoffatome:** 6-Ring Heteroarylgruppen, welche eben Kohlenstoffatomen ein bis drei bzw. ein bis vier Stickstoffatome als Ringglieder enthalten können, z.B. 3-Pyridazinyl, 4-Pyridazinyl, 2-Pyrimidinyl, 4-Pyrimidinyl, 5-Pyrimidinyl, 2-Pyrazinyl, ,3,5-Triazin-2-yl und 1,2,4-Triazin-3-yl;

Ein weiterer Gegenstand er vorliegenden Erfindung betrifft Verfahren zum Herstellen der erfindungsgemäßen Arylpyridazine der Formel (**Ia**) umfassend wenigstens einen der folgenden Schritte (a) bis (d):
(a) Umsetzung von Arylessigsäuren der Formel (**IIb**) mit 2-Haloketonen der Formel (**V**) gemäß nachfolgendem Reaktionsschema: 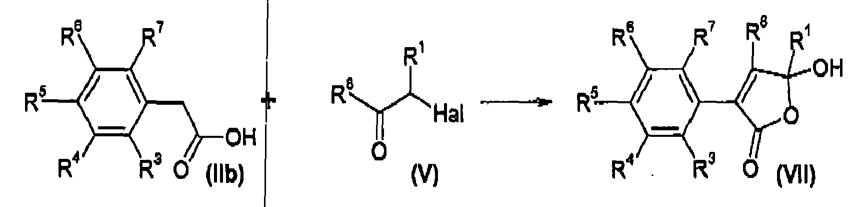
(b) Umsetzung von 2-Acyloxyketonen der Formel (**VI**) gemäß nachfolgendem Reaktionsschema: 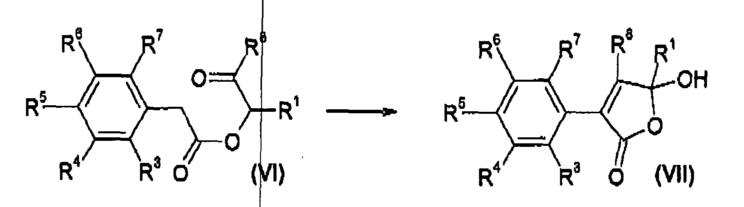
(c) Umsetzung von Hydroxylactonen der Formel (**VII**) mit Hydrazin gemäß nachfolgendem Reaktionsschema; 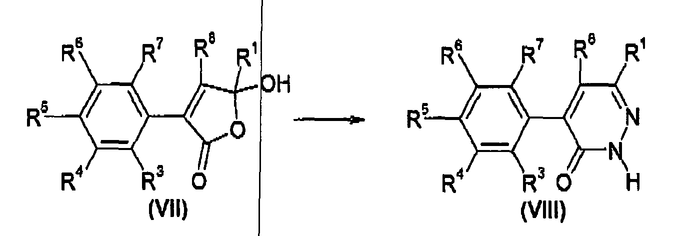
(d) Umsetzung von (2*H*)-Pyridazinonen der Formel (**VIII**) gemäß nachfolgendem Reaktionsschema:

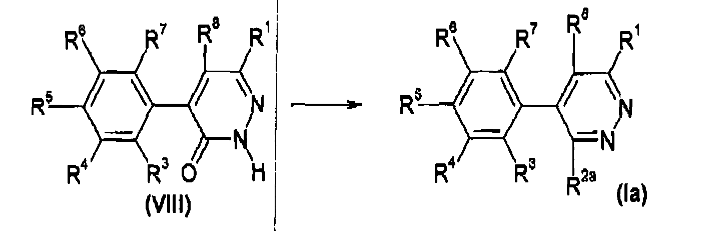

Wobei die Definitionen der Reste R¹ und R³ bis R⁹ in den obigen Schemata den oben angegebenen entsprechen und R^{2a} für Chlor, Brom oder Fluor steht.

Eine Möglichkeit zur Darstellung der erfindungsgemäßen Verbindungen der Formel (**Ia**) ist in Schema 1 gezeigt (siehe dazu beispielsweise auch WO-A1-05/121104),

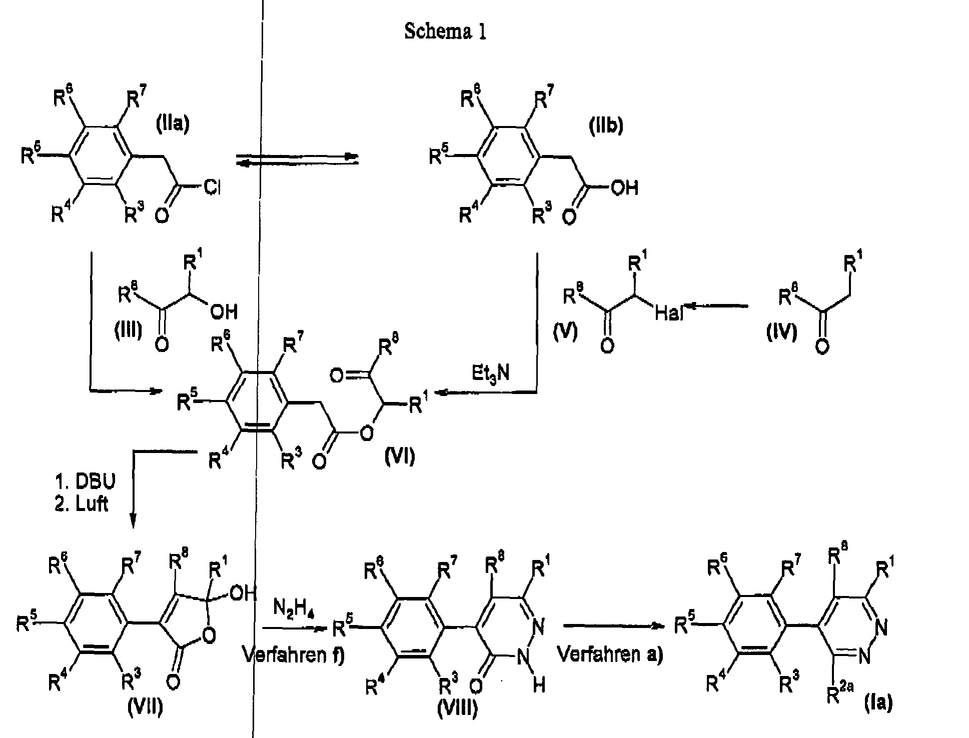

Ausgehend von Arylessigsäurederivaten (**IIa,b**) erhält man durch Umsetzung mit geeigneten substituierten 2-Hydroxyk tonen (**III**) oder 2-Haloketonen (**V**) die 2-Acyloxyketone (**VI**) als Zwischenprodukt. Die zu diesem Zwecke benötigten Verbindungen der Formeln (**IIa,b**), (**III**) und (**V**) sind entweder kommerziell verfügbar oder lassen sich nach literaturbekannten Methoden darstellen. Die Herstellung von Verbindungen der Formel **(III)** ist beispielsweise in Org. Lett. 2005, 7, 5729 oder Chem. Pharm. Bull. 2000, 48, 1338 beschrieben. Eine große Zahl Arylessigsäuren sind in der Literatur beschrieben und/oder kommerziell verfügbar. Darüber hinaus sind Arylessigsäurederivate durch übergangsmetall-katalysierte Reaktionen von Arylhalogeniden (z.B. J. Am. Chem. Soc. 2001, 123, 7996) oder durch Verseifen und Decarboxylierung der entsprechenden Arylmalonsäureester (Synthese z.B. beschrieben in US6156925(2000); J. Org. Chem. 2002, 67, 541; Org, Lett. 2002, 4, 269) zugänglich,

Verbindungen der Formel (**V**) sind durch Halogenierung der entsprechenden Hetarylalkylketone (**IV**) nach literaturbekannte Methoden (z,B, unter Verwendung von Brom, N-Bromsuccinimid, N-Chlorsuccinimid oder Pyridiniumbromid-Perbromid) darstellbar. Die Synthese von geeigneten Hetarylalkylketonen (**IV**) ist literaturbekannt und gelingt beispielsweise durch Friedel-Crafts-Acylierung von Heteroaromaten (z.B. J. Am. Chem. Soc. 1950, 72, 3659) oder durch Reaktion einer Alkyllithium- oder Alkylmagnesiumverbindung mit geeigneten Nitril-, Ester - oder Amidsubstituierten Heteroaromaten.

Die Umsetzung von Verbindungen der Formel (**VI**) in Gegenwart einer starken, nicht nukleophilen Stickstoffbase (z.B, DBU) und anschliessender Luftoxidation *in situ* liefert das korrespondierende Hydroxybutenolid (**VII**). Verbindungen der Formel (**VII**) können nach literaturbekannten Methoden erhalten werden (siehe beispielsweise Synlett 2002, 947; Tetrahedron Lett. 2002, 43, 8715; Indian J. Chem. B. 2003, 42B, 593; Bioorg. Med. Chem. Lett. 2003, 13, 1195); dazu werden beispielsweise geeignet substituierte 2-Acyloxyketone der Formel (VI) in Gegenwart einer starken, nicht nukleophilen Stickstoffbase, beispielsweise DBU, in einem geeigneten Verdünnungsmittel, beispielsweise Acetonitril, zu den korrespondierenden Butenoliden umgesetzt, welche sich bereits in Gegenwart von Umgebungsluft ganz oder teilweise in die Hydroxybutenolide der Formel (VII) umsetzen *(*Bioorg. Med, Chem. Lett. 2003,13, 1195). Zur Vervollständigung der Reaktion wird für einen Zeitraum von ein bis 16 Stunden Sauerstoff, Luft oder ein anderes sauerstoffhaltiges Gas in die Reaktionsmischung eingeleitet. Beide Reaktionsschritte werden einem Temperaturbereich von 0 bis 100°C, vorzugsweise 10 bis 80°C durchgeführt.

Darüber hinaus kann die Reaktionssequenz (**IIb**) → (**VI**)→ (**VII**) auch in einem EintopfVerfahren durchgeführt werden.

Die Umsetzung von Hydroxybutenoliden der Formel (**VII**) mit Hydrazin gemäß Verfahren **f**) ergibt die korrespondierenden Pyridazinone der Formel (**VIII**) (siehe Schema 2). Zur Durchführung von Verfahren **f**) setzt man die Verbindungen der Formel (**VII**) mit Hydrazinhydrat in einem geeigenten Verdünnungsmittel bei Temperaturen zwischen 20°C und 180°C um. Bevorzugt kommen dabei als Verdünnungsmittel Alkohole wie zum Beispiel Methanol, Ethanol, Propanol, Butanol oder Ethylenglyko in Frage.

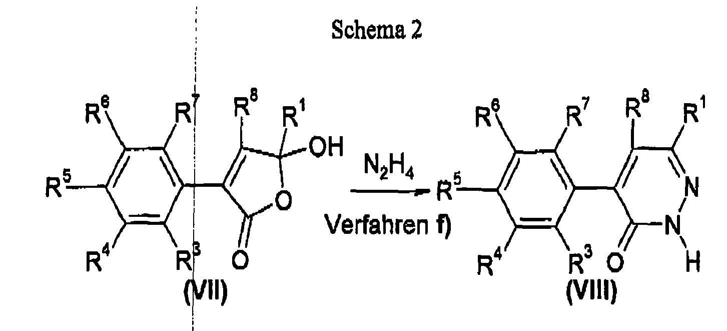

Hydroxybutenolide der Formel (**VII**), bei denen R¹ und R³ bis R⁹ die oben angegebenen Bedeutungen haben, sowie deren Umsetzung in die korrespondierenden Verbindungen der Formel (**VIII**) gemäß Verfahren **f**) sind neu und daher ebenfalls Gegenstand der Erfindung.

Verbindungen der Formel (**VIII**) können durch Reaktion mit einem geeigneten Halogenierungsmittel gemäß Verfahren a) in die korrespondierenden 3-halogensubstituierten Pyridazine (**Ia**) überführt werden,

Verbindungen der Formel (**VII**) werden erhalten, indem man
i) Verbindungen der Forme (**IIb**) 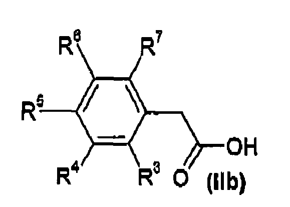 mit Verbindungen der Formel (**V**) 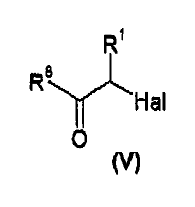 in welcher
   R¹ und R³ bis R⁹ die oben angegebenen Bedeutungen haben gegebenenfalls in Gegenwart eines Säureakzeptors gegebenfalls in Gegenwart eines Verdünnungsmittels umsetzt oder alternativ
ii) Verbindungen der Form (**VI**) 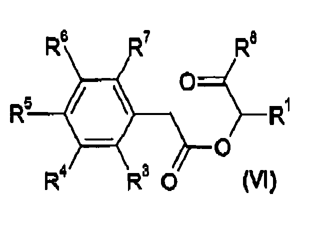 in welcher
   R¹ und R³ bis R⁹ jeweils die oben angegebenen Bedeutungen haben gegebenenfalls in Gegenwart einer Base in Gegenwart von Luft gegebenenfalls in Gegenwart eines Verdünnungsmittels umsetzt
      Verbindungen der Formel (**VIII**)werden erhalten, indem man
iii) Verbindungen der Formel (**VII**) 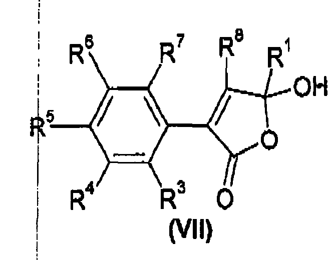
in welcher
R¹ bis R⁹ die oben angegebenen Bedeutungen haben mit Hydrazin oder Hydrazinhydrat gegebenenfalls in Gegenwart eines Verdünnungsmittels umsetzt.

Als Halogenierungsmittel Kommen bei der Durchführung des erfindungsgemäßen Verfahrens **a**) alle für den Ersatz von Hydroxygruppen durch Halogen üblichen Substanzen in Betracht. Vorzugsweise verwendbar sind Phosphortrichlorid, Phosphortribromid, Phosphorpentachlorid, Phosphoroxychlorid, Thionylchlorid, Thionylbromid oder deren Gemische oder Phosgen, Di- oder Triphosgen. Gegebenenfalls wird Chlor zu den genannten Halogenierungsmitteln oder ihren Mischungen gegeben. 3-Fluorpyridazine der Formel (**Ia**) (R^{2a}= F) lassen sich aus den Chlor- oder Brom-Verbindungen durch Umsetzung mit einer Fluoridquelle, beispielsweise Kaliumfluorid, ggf. in Gegenwart eines Katalysators, beispielsweise Tetraphenylphosphoniumbromid, herstellen. Als Lösungsmittel kann beispielsweise Sulfolan verwendet werden.

Die Reaktion wird üblicherweise in einem Temperaturbereich von 20 bis 180°C durchgeführt.

Als Verdünnungsmittel kommen bei der Durchführung des erfindungsgemäßen Verfahrens alle für derartige Halogenierunge üblichen Solventien in Frage. Vorzugsweise verwendbar sind halogenierte aliphatische der aromatische Kohlenwasserstoffe wie beispielsweise Chlorbenzol.

Als Verdünnungsmittel kann aber auch das Halogenierungsmittel selbst, z.B. Phosphoroxychlorid oder ein Gemisch von Halogenierungsmitteln fungieren.

Als Säureakzeptoren kommen bei der Durchführung des erfindungsgemäßen Verfahrens a) alle für derartige Umsetzungen üblichen anorganischen und organischen Basen in Frage. Vorzugsweise verwendbar sind Erdalkalimetall- oder Alkalimetallhydroxide, -acetate, -carbonate, -hydrogencarbo-nate oder Phosphate, wie beispielsweise Natriumhydroxid, Kaliumhydroxid, Natriumacetat Natriumcarbonat, Kaliumcarbonat, Kaliumhydrogencarbonat, Natriumhydrogencarbonat, Cäsiumcarbonat oder Silberphosphat, sowie tertiäre Amine wie beispielsweise N,N-Diethylanilin oder Ethyldiisopropylamin.

Die Temperaturen können auch bei der Durchführung des Verfahrens **a**) in einem größeren Bereich variiert werden. Im Allgemeinen arbeitet man bei Temperaturen zwischen 0°C und 150°C, vorzugsweise zwischen 10° und 120°C,

Bei der Durchführung des Verfahrens **a**) setzt man die Verbindung der Formel (**VIII**) im Allgemeinen mit einem Überschuss an Halogenierungsmittel um. Die Aufarbeitung erfolgt nach üblichen Methoden,

(2*H*)-Pyridazinone der Formel (**VIII**), bei denen R¹ und R³ bis R⁹ die oben angegebenen Bedeutungen haben, sowie deren Umsetzung in die korrespondierenden Verbindungen der Formel (**Ia**) gemäß Verfahren a) (siehe Schema 3) sind neu und daher ebenfalls Gegenstand der Erfindung.

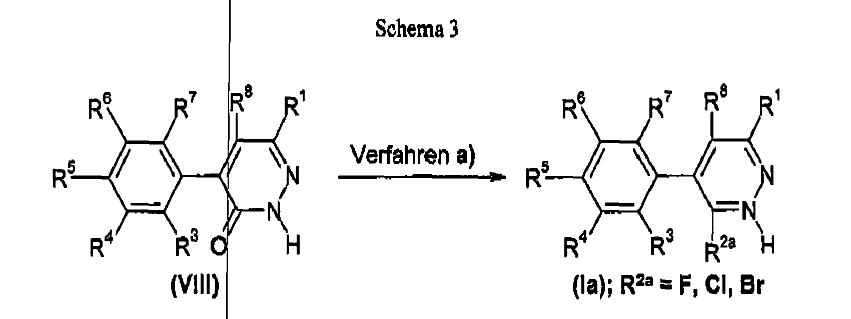

Die Pyridazine der Formel (**Ia**) werden erhalten, indem man
iiii) (2H)-Pyridazinone der Formel (**VIII**) 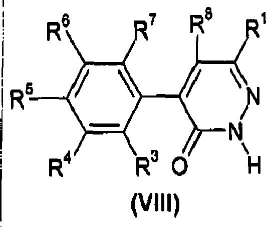 in welcher
   R¹ bis R⁹ die oben angegebenen Bedeutungen haben mit einem Halogenierungsmittel gegebenenfalls in Gegenwart eines Säureakzeptors und gegebenenfalls in Gegenwart eines Verdünnungsmittels umsetzt. Gegebenenfalls werden die so erhaltenen Chlor- oder Brompyridazine in einer Folgestufe mit einer Fluoridquelle, beispielsweise Kaliumfluorid oder Tetraethylammoniumfluorid gegebenenfalls in Gegenwart eines Katalysators, beispielsweise Tetraphenylphosphoniumbromid, und gegebenenfalls in Gegenwart eines Verdünnungsmittels, beispielsweise Sulfolan, weiter umgesetzt.

Pyridazine der Formel (**Ia**), bei denen R¹ bis R⁷ die oben angegebenen Bedeutungen haben, sowie deren Umsetzung in die korrespondierenden Verbindungen der Formel (**Ib**) gemäß Verfahren **b**) (siehe Schema 4) sowie deren Umsetzung in die korrespondierenden Verbindungen der Formel (**Ic**) gemäß Verfahren c) (siehe Schema 4) sind neu und daher ebenfalls Gegenstand der vorliegenden Erfindung.

Ebenso ist die Umsetzung der Pyridazine der Formel (**Ic**) in die korrespondierenden Verbindungen der Formel (**Id**) gemäß Verfahren d) (siehe Schema 4) neu und daher ebenfalls Gegenstand der vorliegenden Erfindung, 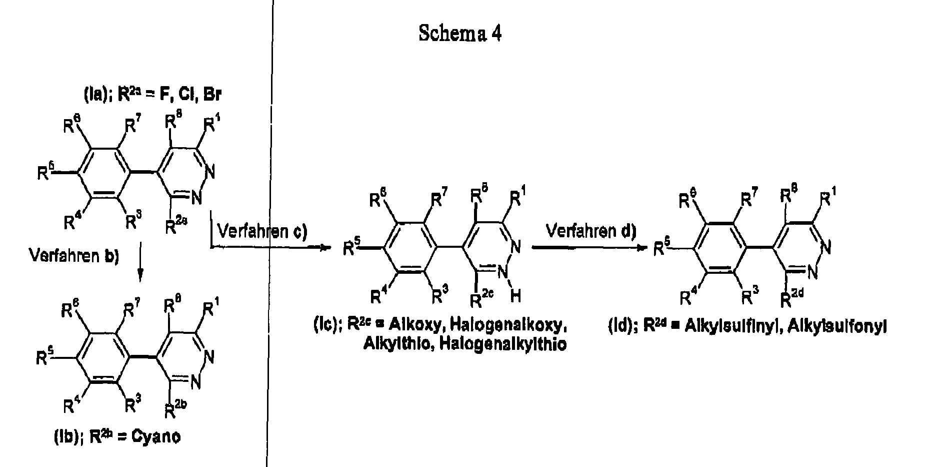 3-Halogensubstituierte Pyridazine der Formel (**Ia**) lassen sich in einem Folgeschritt nach literaturbekannten Methoden gemäß Verfahren **b**) durch nukleophile Substitution in die korrespondierenden 3-Cyano-substituierten Pyridazine der Formel **(Ib)** (R^{2b}= CN) überführen (siehe Schema 4). Die Einführung der Cyanogruppe gelingt beispielsweise durch die Reaktion von z.B. einem Alkalicyanid, z.B. Kalium- oder Natriumcyanid, oder einer alternativen Cyanidquelle wie beispielsweise Kupfercyanid, Zinkcyanid, Trimethylsilylcyanid oder K₄[Fe(CN₆)] in einem geeigneten Lösungsmittel, beispielsweise oder Dimethylsulfoxid, in einem Temperaturbereich von 20 bis 150°C, Alternativ ist es möglich, die Reaktion in Gegenwart eines Übergangsmetallkatalysators durchzuführen (z.B. beschrieben in Tetrahedron Lett. 2007, 48, 1087; DE-A 05/102005009517)

Die Pyridazine der Formel (**Ib**) werden erhalten, indem man
iiiii) Pyridazine der Formel (**Ia**) 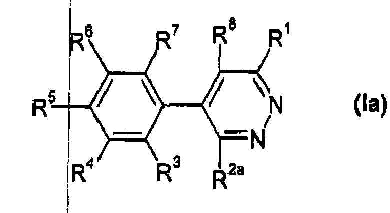 in welcher
   R¹ und R³ bis R⁹ die oben angegebenen Bedeutungen haben und R^{2a} für F, Cl oder Br steht mit einer Cyanidquelle, geben falls in Gegenwart eines Säureakzeptors, gegebenenfalls in Gegenwart eines Übergangsmetallkatalysators und gegebenenfalls in Gegenwart eines Verdünnungsmittel umsetzt.

Die zur Durchführung des erfindungsgemässen Verfahrens b) als Ausgangsstoffe benötigten Pyridazine der Formel (**Ia**) wurden bereits oben beschrieben.

Bestimmte 3-Alkoxy-, 3-Halogenalkoxy 3-Alkylthio- oder 3-Halogenalkylthio-substituierte Pyridazine der Formel (**Ic**) (R^{2c}= Alkoxy, Halogenalkoxy, Alkylthio, Halogenalkylthio) sind in einem Folgeschritt gemäß Verfahren c) durch nukleophile Substitution aus den korrespondierenden 3-Halogen-substituierten Verbindungen der Formel (**Ia**) (R^{2a} = Fluor, Chlor, Brom) darstellbar (siehe Schema 4). Zu diesem Zweck versetzt man setzt man mit entsprechenden Alkoholen, beispielsweise Methanol, Isopropanol oder 2-Methoxyethanol, oder Thioalkoholen, beispielsweise Methanthiol oder Ethanthiol, in Gegenwart von typischen organischen oder anorganischen Basen, beispielsweise Kaliumcarbonat, Kalium-tert-butylat oder Natriumhydrid, in einem geeigneten Lösungsmittel, beispielsweise Tetrahydrofuran, 1,4-Dioxan, N,N-Dimethyformamid oder Dimethylsulfoxid, in einem Temperaturbereich von 20°C bis 150°C um.

Die Pyridazine der Formel (**Ic**) werden erhalten, indem man
iiiiii) Pyridazine der Formel (**Ia**) 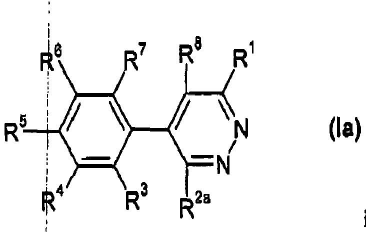 in welcher
   R¹ und R³ bis R⁹ die oben angegebenen Bedeutungen haben und R^{2a} für F, Cl oder Br steht mit Alkoholen oder Thioalkoholen in Gegenwart von Basen in einem Lösungsmittel umsetzt.

Die zur Durchführung des erfindungsgemässen Verfahrens c) als Ausgangsstoffe benötigten Pyridazine der Formel (**Ia**) wurden bereits oben beschrieben.

Bestimmte 3-Alkylthio- oder 3-Halogenalkylthio-substituierte Pyridazine der Formel (**Ic**) (R^{2c} = Alkylthio, Halogenalkylthio) lassen sich durch Oxidation mit organischen Persäurederivaten, ggf. in Gegenwart eines Katalysators wie z,B, Ammoniummolybdat(VI) (NH₄)₂MoO₄, in geeigneten Lösungsmitteln wie beispiel;weise Dichlormethan oder Trichlormethan oder Gemischen davon mit Wasser gemäß Verfahren **d**) in die korrespondierenden 3-Alkylsulfinyl- oder 3-Halogenalkylsulfinyl-Pyridazine bzw, 3-Alkylsulfonyl- oder Halogenalkylsulfonylpyridazine der Formel (**Id**) (R^{2d}= Alkylsulfinyl, Halogenalkylsulfinyl, Alkylsulfonyl, Halogenalkylsulfonyl) überführen (siehe Schema 4). Die in Frage kommenden Persäurederivate wie beispielsweise *m-*Chlorperbenzoesäure, Magnesiummonoperoxyphthalat, Peressigsäure oder Trifluorperessigsäure sind entweder kommerziell verfügbar oder werden *in situ* aus den korrespondierenden Carbonsäuren (z.B. Essigsäure oder Trifluoressigsäure) in Gegenwart von Wasserstoffperoxid erzeugt. Darüber hinaus kann die Reaktion ggf. in Gegenwart eines geeigneten Säureakzeptors wie beispielsweise Natriumcarbonat durchgeführt werden.

Die Temperaturen können bei der Durchführung des Verfahrens **d**) in einem größeren Bereich variiert worden. Im Allgemeinen arbeitet man bei Temperaturen zwischen 0°C und 100°C, vorzugsweise zwischen 10°C und 70°C.

Die Pyridazine der Formel (**Id**) werden erhalten, indem man
iiiiiii) Pyridazine der Formel (**Ic**) 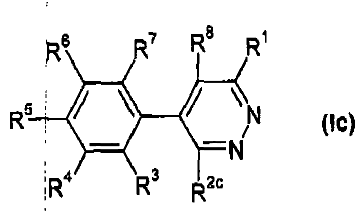 in welcher
   R¹ und R³ bis R⁹ die oben angegebenen Bedeutungen haben und R²⁰ für Alkoxy, Halogenalkoxy, Alkylthio, Halogenalkylthio steht mit Oxidationsmitteln gegebenenfalls in Gegenwart eines Katalysators, gegebenenfalls in Gegenwart eines Säureakzeptors und gegebenenfalls in Gegenwart eines Lösungsmittels umsetzt.

Die zur Durchführung des erfindungsgemässen Verfahrens **d**) als Ausgangsstoffe benötigten Pyridazine der Formel (**Ic**) wurden bereits oben beschrieben.

Die erfindungsgemäßen Verfahren zur Herstellung der Verbindungen der Formeln (**Ia**) bis (**Id**) werden vorzugsweise unter Verwendung eines oder mehrerer Reaktionshilfsmittel durchgeführt.

Als Reaktionshilfsmittel kommen gegebenenfalls die üblichen anorganischen oder organischen Basen oder Säureakzeptoren in Betracht. Hierzu gehören vorzugsweise Alkalimetall- oder Erdalkalimetall- -acetate, -amide, -carbonate, -hydrogencarbonate, -hydride, -hydroxide oder -alkanolate, wie beispielsweise Natrium-, Kalium- oder Calcium-acetat, Lithium-, Natrium-, Kalium- oder Caleium-amid, Natrium-, Kalium- oder Calciunn-carbonat, Natrium-, Kalium- oder Calcium-hydrogencarbonat, Lithium-, Natrium-, Kalium- oder Calcium-hydrid, Lithium-, Natrium- - Kalium- oder Calcium-hydroxid, Natrium- oder Kalium- -methanolat, -ethanolat, -n- oder -i-propanolat, -n-, -i-, -s- oder -tButanolat; weiterhin auch basische organische Stickstoffverbindungen, wie beispielsweise Trimethylamin, Triethylamin, Tripropylamin, Tributylamin, Ethyldiisopropylamin, N,N-Dimethyl-cyclohexylamin, Dicyclohexylamin, Ethyl-dicycloboxylamin, N,N-Dimethyl-anilin, N,N-Dimethyl-benzylamin, Pyridin, 2-Methyl-, 3-Methyl-, 4-Methyl-, 2,4-Dimethyl-, 2,6-Dimethyl-, r3,4-Dimethyl-und 3,5-Dimethyl-pyridin, 5->Jthyl-2-methyl-pyridin, 4-Dimethylamino-pyridin,N-Methyl-piperidin, 1,4-Diazabicyclo[2,2,2]-ortan (DABCO), 1,5-Diaza-bicyclo[4,3,0]-non-5-en (DBN), oder 1,8 Diazabicyclo[5,4,0]-undec-7-en (DBU).

Als weitere Reaktionshilfsmittel kommen gegebenenfalls die üblichen anorganischen oder organischen Brönstedt- bzw. Lowis-Säuren in Betracht. Hierzu gehören vorzugsweise Halogenwasserstoffsäuren oder Mineralsäuren wie beispielsweise Chlor- oder Bromwasserstoffsäure, Schwefelsäure, schweflige Säure, Salpetersäure, salpetrige Säure, Phosphorsäure, Lewis-Säuren wie beispielsweise Aluminiumtrichlorid, Bortrifluorid, Bortrichlorid, Bortribromid, Titantetrachlorid, Zinntetrachlorid, Certrichlorid, (Übergangs-) Metalltriflate wie beispielsweise Trialkylsilyltriflate, Kupfertriflat, Ytterbiumtriflat, Imide wie beispielsweise Trifluormethansulfonimid, Sulfonsäuren wie beispielsweise Methansulfonsäure, Trifluormethansulfonsäure, p-Toluolsulfonsäure, Camphersulfonsäure, Carbonsäuren wie beispielsweise Ameisensäure, Essigsäure, Trifluoressigsäure, Propionsäure, Oxalsäure, Benzoesäure.

Die erfindungsgemäßen Verfahren werden vorzugsweise unter Verwendung eines oder mehrerer Verdünnungsmittel durchgeführt. Als Verdünnungsmittel kommen praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl-und Dibutylether, Glykoldimethylether und Diglykoldimethylether, Tetrahydrofuran und Dioxan, Ketone wie Aceton, Methyl-ethyl-, Methyl-isopropyl-oder Methyl-isobutyl-keton, Ester wie Essigsäuremethylester oder-ethylester, Nitrile wie z.B. Acetonitril oder Propionitril, Amide wie z,B. Dimethylformamid, Di-methylacetamid und N-Methyl-pyrrolidon, sowie Dimethylsulfoxid, Tetramethylensulfon und Hexamethylphosphorsäurenfarnid und DMPU.

Die Reaktionstemperaturen! können bei den erfindungsgemäßen Verfahren in einem größeren Bereich variiert werden. Im allgemeinen arbeitet man bei Temperaturen zwischen 0°C und 250°C, vorzugsweise bei Temperaturen zwischen 10°C und 185°C.

Die erfindungsgemäßen Verfahren werden im allgemeinen unter Normaldruck durchgeführt. Es ist jedoch auch möglich, unter erhöhtem oder vermindertem Druck zu arbeiten.

Zur Durchführung der erfindungsgemäßen Verfahren werden die jeweils benötigten Ausgangsstoffe im allgemeinen in angenähert äquimolaren Mengen eingesetzt. Es ist jedoch auch möglich, eine der jeweils eingesetzten Komponenten in einem größeren Überschuss zu verwenden. Die Aufarbeitung erfolgt bei den erfindungsgemäßen Verfahren jeweils nach üblichen Methoden (vgl. die Herstellungsbeispiele).

Die erfindungsgemäßen Stoffe weisen eine starke mikrobizide Wirkung auf und können zur Bekämpfung von unerwünschten Mikroorganismen, wie Fungi und Bakterien, im Pflanzenschutz und im Materialschutz eingesetzt werden,

Fungizide lassen sich im Pflanzenschutz beispielsweise zur Bekämpfung von Plasmodiophoromycetes, Oomycetes, Chytridiomycetes, Zygomycetes, Ascomycetes, Basidiomycetes und Deuteromycetes einsetzen.

Bakterizide lassen sich im Pflanzenschutz beispielsweise zur Bekämpfung von Pseudomonadaceae, Rhizobiaceae, Enterobacteriaceae, Corynobacteriaceae und Streptomycetaceae einsetzen.

Die erfindungsgemäßen Arylpyridazine besitzen sehr gute fungizide Eigenschaften und lassen sich zur Bekämpfung von phytopathogenen Pilzen, wie Plasmodiophoromycetes, Oomycetes, Chytridiomycetes, Zygomycetes, Ascomycetes, Basidiomycetes, Deuteromycetes usw. einsetzen.

Beispielhaft aber nicht begrenzend seien einige Erreger von pilzlichen und bakteriellen Erkrankungen, die unter die oben aufgezählten Oberbegriffe fallen, genannt:
Xanthomonas-Arten, wie beispielsweise Xanthomonas campestris pv, oryzae;
Pseudomonas-Arten, wie beispielsweise Pseudomonas syringae pv. lachrymans;
Erwinia-Arten, wie beispielsweise Erwinia amylovora;
Erkrankungen, hervorgerufen durch Erreger des Echten Mehltaus wie z.B.
Blumeria-Arten, wie beispielsweise Blumeria graminis;
Podosphaera-Arten, wie beispielsweise Podosphaera leucotricha;
Sphaerotheea-Arten, wie beispielsweise Sphaerotheca fuliginea;
Uncinula-Arten, wie beispielsweise Uncinula necator;
Erkrankungen, hervorgerufen durch Erreger von Rostkrankheiten wie z.B.
Gymnosporangium-Arten, wie beispielsweise Gymnosporangium sabinae
Hemileia-Arten, wie beispielsweise Hemileia vastatrix;
Phakopsora-Arten, wie beispielsweise Phakopsora pachyrhizi und Phakopsora meibomiae;
Puccinia-Arten, wie beispielsweise Puccinia recondita;
Uromyces-Arten, wie beispielsweise Uromyces appendiculatus;
Erkrankungen, hervorgerufen durch Erreger der Gruppe der Oomyceten wie z.B.
Bremia-Arten, wie beispielsweise Bremia lactucae;
Peronospora-Arten, wie beispielsweise Peronospora pisi oder P. brassicae;
Phytophthora-Arten, wie beispielsweise Phytophthora infestans;
Plasmopara-Arten, wie beispielsweise Plasmopara viticola;
Pseudoperonospora-Arten, wie beispielsweise Pseudoperonospora humuli oder
Pseudoperonospora cubensis;
Pythium-Arten, wie beispielsweise Pythium ultimum;
Blattfleckenkrankheiten und Blattwolken, hervorgerufen durch z.B.
Alternaria-Arten, wie beispielsweise Alternaria solani;
Cercospora-Arten, wie beispielsweise Cercospora beticola;
Cladiosporum-Arten, wie beispielsweise Cladiosporium cucumerinum;
Cochliobolus-Artcn, wie beispielsweise Cochliobolus sativus
(Konidienform: Drechslera, Syn: Helminthosporium);
Colletotrichum-Arten, wie beispielsweise Colletotrichum lindemuthanium;
Cycloconium-Arten, wie beispielsweise Cycloconium oleaginum;
Diaporthe-Arten, wie beispielsweise Diaporthe citri;
Elsinoe-Arten, wie beispielsweise Elsinoe fawcettii;
Gloeosporium-Arten, wie beispielsweise Gloeosporium laeticolor;
Glomerella-Arten, wie beispielsweise Glomerella cingulata;
Guignardia-Arten, wie beispielsweise Guignardia bidwelli;
Leptosphaeria-Arten, wie beispielsweise Leptosphaeria maculans;
Magnaporthe-Arten, wie beispielsweise Magnaporthe grisea;
Mycosphaerella-Arten, wie beispielsweise Mycosphaerella graminicola und Mycosphaerella fijiensis;
Phaeosphaeria-Arten, wie beispielsweise Phaeosphaeria nodorum;
Pyrenophora-Arten, wie beispielsweise Pyrenophora teres;
Ramularia-Arten, wie beispielsweise Ramularia collo-cygni;
Rhynchosporium-Arten, wie beispielsweise Rhynchosporium secalis;
Septoria-Arten, wie beispielsweise Septoria apii;
Typhula-Arten, wie beispielsweise Typhula incarnata;
Venturia-Artcn, wie beispielsweise Venturia inaequalis;
Wurzel-und Stengelkrankheiten, hervorgerufen durch z.B.
Corticium-Arten, wie beispielsweise Corticium graminearum;
Fusarium-Arten, wie beispielsweise Fusarium oxysporum;
Gaeuma.nnomyces-Arten, wie beispielsweise Gaeumannomyces graminis;
Rhiaoctonia-Arten, wie beispielsweise Rhizoctonia solani;
Tapesia-Arten, wie beispielsweise Tapesia acuformis;
Thiolaviopsis-Arten, wie bei spielsweise Thielaviopsis basicola;
Ähren-und Rispenerkrankungen (inklusive Maiskolben), hervorgerufen durch z.B.
Alternaria-Arten, wie beispielsweise Alternaria spp.;
Aspergillus-Arten, wie beispielsweise Aspergillus flavus;
Cladosporium-Arten, wie beispielsweise Cladosporium spp, cladosporioides;
Claviceps-Arten, wie beispielsweise Claviceps purpurea;
Fusarium-Arten, wie beispielsweise Fusarium culmorum;
Gibberella-Arten, wie beispielsweise Gibberella zeae;
Monographella-Arten, wie beispielsweise Monographella nivalis;
Erkrankungen, hervorgerufen durch Brandpilze wie z.B.
Sphacelotheca-Arten, wie beispielsweise Sphacelotheca reiliana;
Tilletia-Arten, wie beispielsweise Tilletia caries;
Urocystis-Arton, wie beispielsweise Urocystis occulta;
Ustilago-Arten, wie beispielsweise Ustilago nuda;
Fruchtfäule hervorgerufen durch z.B.
Aspergillus-Arten, wie beispielsweise Aspergillus flavus;
Botrytis-Arten, wie beispielsweise Botrytis cinerea;
Penicillium-Arten, wie beispielsweise Penicillium expansum und Penicillium purpurogenum;
Sclerotinia-Arten, wie beispielsweise Sclerotinia sclerotiorum;
Verticilium-Arten, wie beispielsweise Verticilium alboatrum;
Samen-und bodenbürtige Fäulen und Welken, sowie Sämlingserkrankungen, hervorgerufen durch z.B.
Fusarium-Arten, wie beispielsweise Fusarium culmorum;
Phytophthora Arten, wie beispielsweise Phytophthora cactorum;
Pythium-Arten, wie beispielsweise Pythium ultimum;
Rhizootonia-Arten, wie beispielsweise Rhizoctonia solani;
Sclerotium-Arten, wie beispielsweise Sclerotium rolfsii;
Krebserkrankungen, Gallen und Hexenbesen, hervorgerufen durch z.B.
Nectria-Arten, wie beispielsweise Nectria galligena;Welkeerkrankungen hervorgerufen durch z.B.
Monilinia-Arten, wie beispielsweise Monilinia laxa;
Deformationen von Blättern, Blüten und Früchten, hervorgerufen durch z.B.
Taphrina-Arten, wie beispielsweise Taphrina deformans;
Degenerationserkrankungen holziger pflanzen, hervorgerufen durch z.B.
Esca-Arten, wie beispielsweise Phaeomoniella chlamydospora und Phaeoacremonium aleophilum und Fomitiporia mediterranea;
Blüten-und Samenerkrankungen, hervorgerufen durch z.B.
Botrytis-Arten, wie beispielsweise Botrytis cinerea;
Erkrankungen von Pflanzenknollen, hervorgerufen durch z.B.
Rhizoctonia-Arten, wie beispielsweise Rhizoctonia solani;
Erkrankungen, hervorgerufen durch bakterielle Erreger wie z.B.
Xanthomonas-Arten, wie beispielsweise Xanthomonas campestris pv. oryzae;
Pseudomonas-Arten, wie beispielsweise Pseudomonas syringae pv. lachrymans;
Erwinia-Arten, wie beispielsweise Erwinia amylovora;
Bevorzugt können die folgenden Krankheiten von Soja-Bohnen bekämpft werden:
Pilzkrankheiten an Blättern, Stängeln, Schoten und Samen verursacht durch z.B.

Alternaria leaf spot (Alternaria spec. atrans tenuissima), Anthracnose (Colletotrichum gloeosporoides dematium var. truncatum), Brown spot (Septoria glycines), Cercospora leaf spot and blight (Cercospora kik-üchii), Choanephora leaf blight (Choanephora infundibulifera trispora (Syn.)), Dactuliophora leaf spot (Dactuliophora glycines), Downy Mildew (Peronospora manshurica), Drechslera blight (Drechslera glycini), Frogeye Leaf spot (Cercospora sojina), Leptosphaerulina Leaf Spot (Leptosphaerulina trifolii), Phyllostica Leaf Spot (Phyllosticta sojaecola), Pod and Stem Blight (Phomopsis sojae), Powdery Mildew (Microsphaera diffusa), Pyrenochaeta Leaf Spot (Pyrenochaeta glycines), Rhizoctonia Aerial, Foliage, and Web Blight (Rhizoctonia solani), Rust (Phakopsora pachyrhizi), Scab (Sphaceloma glycines), Stemphylium Leaf Blight (Stemphylium botryosum), Target Spot (Corynespora cassücola),

Pilzkrankheiten an Wurzeln und der Stängelbasis verursacht durch z.B.

Black Root Rot (Calonectria crotalariae), Charcoal Rot (Macrophomina phasoolina), Fusarium Blight or Wilt, Root Rot, and Pod and Collar Rot (Fusarium oxysporum, Fusarium orthoceras, Fusarium semitectum, Fusarium equiseti), Mycoleptodiscus Root Rot (Mycoleptodiscus terrestris), Neocosmospora (Neocosmopspora vasinfecta), Pod and Stem Blight (Diaporthe phaseolorum), Stem Canker (Diaporthe phaseolorum var. caulivora), Phytophthora Rot (Phytophthora megasperma), Brown Stem Rot (Phialophora gregata), Pythium Rot (Pythium aphanidermatum, Pythium irregulare, Pythium debaryanum, Pythium myriotylum, Pythium ultimum), Rhizoctonia Root Rot, Stem Decay, and Damping-Off (Rhizoctonia solani), Sclerotinia Stem Decay (Sclerotinia sclerotiorum), Sclerotinia Southern Blight (Sclerotinia rolfsii), Thielaviopsis Root Rot (Thielaviopsis basicola).

Die erfindungsgemäßen Wirkstoffe weisen auch eine sehr gute stärkende Wirkung in Pflanzen auf. Sie eignen sich daher zur Mobilisierung pflanzeneigener AbwehrkräAe gegen Befall durch unerwünschte Mikroorganismen.

Unter pflanzenstärkenden (resistenzinduzierenden) Stoffen sind im vorliegenden Zusammenhang solche Substanzen zu verstehen, die in der Lage sind, das Abwehrsystem von Pflanzen so zu stimulieren, dass die behandelten Pflanzen bei nachfolgender Inokulation mit unerwünschten Mikroorganismen weitgehende Resistenz gegen diese Mikroorganismen entfalten.

Unter unerwünschten Mikroorganismen sind im vorliegenden Fall phytopathogene Pilze und Bakterien zu verstehen. Die erfindungsgemäßen Stoffe können also eingesetzt werden, um Pflanzen innerhalb eines gewissen Zeitraumes nach der Behandlung gegen den Befall durch die genannten Schaderreger zu schützen. Der Zeitraum, innerhalb dessen Schutz herbeigeführt wird, erstreckt sich im allgemeinen von 1 bis 10 Tage, vorzugsweise 1 bis 7 Tage nach der Behandlung der Pflanzen mit den Wirkstoffen.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz-und Saatgut, und des Bodens.

Dabei lassen sich die erfindungsgemäßen Wirkstoffe mit besonders gutem Erfolg zur Bekämpfung von Getreidekrankheiten, wie beispielsweise gegen Erysiphe-Arten, gegen Puccinia und gegen Fusarien-Arten, von Reiskrankheiten, wie beispielsweise gegen Pyricularia und Rhizoctonia und von Krankheiten im Wein-, Obst-und Gemüseanbau, wie beispielsweise gegen Botrytis-, Venturia-, Sphaerotheca-und Podosphaera-Arten, einsetzen.

Die erfindungsgemäßen Wirkstoffe eignen sich auch zur Steigerung des Ernteertrages. Sie sind außerdem mindertoxisch und weisen eine gute Pflanzenverträglichkeit auf.

Die erfindungsgemäßen Wirkstoffe können gegebenenfalls in bestimmten Konzentrationen und Aufwandmengen auch als Herbizide, zur Beeinflussung des Pflanzenwachstums, sowie zur Bekämpfung von tierischen Schädlingen verwendet werden. Sie lassen sich gegebenenfalls auch als Zwischen-und Vorprodukte für die Synthese weiterer Wirkstoffe einsetzen.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs-und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Spross, Blatt, Blüte und Wurzel verstanden werden, wobei beispielhaft Blätter, Nadeln, Stängel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen und bei Vermehrungsmaterial, insbesondere bei Samen, weiterhin durch ein-oder mehrschichtiges Umhüllen.

Darüber hinaus kann durch die erfindungsgemäße Behandlung der Mykotoxingehalt im Erntegut und den daraus hergestellten Nahrungs- und Futtermitteln verringert werden, Besonders, aber nicht ausschließlich sind hierbei folgende Mykotoxine zu nennen: Deoxynivalenol (DON), Nivalenol, 15-Ac-DON, 3-Ac-DON, T2- und HT2- Toxin, Fumonisine, Zearalenon, Moniliformin, Fusarin, Diaceotoxyscirpenol (DAS), Beauvericin, Enniatin, Fusaroproliferin, Fusaronol, Ochratoxine, Patulin, Mutterkornalkaloide und Aflatoxine, die beispielsweise von den folgenden Pilzen verursacht werden können: Fusarium spec., wie Fusarium acuminatum, F. avenaceum, F. crookwellense, F. culmorum, F. graminearum (Gibberella zeae), F. equiseti, F. fujikoroi, F. musarum, F. oxysporum, F. proliferatum, F, poae, F. pseudograminearum, F. sambucinum, F. scirpi, F. semitectum, F. solani, F. sporotrichoides, F. langsethiae, F. subglutinans, F, tricinctum, F. verticillioides u.a, sowie auch von Aspergillus spec., Penicillium spec., Claviceps purpurea, Stachybotrys spec. u.a.

Im Materialschutz lassen sich die erfindungsgemäßen Stoffe zum Schutz von technischen Materialien gegen Befall und Zerstörung durch unerwünschte Mikroorganismen einsetzen.

Unter technischen Materialien sind im vorliegenden Zusammenhang nichtlebende Materialien zu verstehen, die für die Verwendung in der Technik zubereitet worden sind. Beispielsweise können technische Materialien, die durch erfindungsgemäße Wirkstoffe vor mikrobieller Veränderung oder Zerstörung geschützt werden sollen, Klebstoffe, Leime, Papier und Karton, Textilien, Leder, Holz, Anstrichmittel und Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen, beispielsweise Kühlwasserkreisläufe, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiermittel und Wärmeübenragmgsflüssigkeiten genannt, besonders bevorzugt Holz.

Als Mikroorganismen, die einen Abbau oder eine Veränderung der technischen Materialien bewirken können, seien beispielsweise Bakterien, Pilze, Hefen, Algen und Schleimorganismen genannt.

Vorzugsweise wirken die erfindungsgemäßen Wirkstoffe gegen Pilze, insbesondere Schimmelpilze, holzverflärbende und holzzerstörende Pilze (Basidiomyceten) sowie gegen Schleimorganismen und Algen.

Es seien beispielsweise Mikroorganismen der folgenden Gattungen genannt:
Alternaria, wie Alternaria tenuis,
Aspergillus, wie Aspergillus niger,
Chaetomium, wie Chaetomium globosum,
Coniophora, wie Coniophora puetana,
Lentinus, wie Lentinus tigrinus,
Penicillium, wie Penicillium glaucum,
Polyporus, wie Polyporus versicolor,
Aureobasidium, wie Aureobasidium pullulans,
Sclerophoma, wie Sclerophoma pityophila,
Trichoderma, wie Trichoderma viride,
Escherichia, wie Escherichia coli,
Pseudomonas, wie Pseudomonas aeruginosa,
Staphylococcus, wie Staphylococcus aureus.

Die Wirkstoffe können in Abhängigkeit von ihren jeweiligen physikalischen und/ oder chemischen Eigenschaften in die üblichen Formulierungen überführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt-und Warmnebel-Formulierungen,

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln, Im Falle der Benutzung von Wasser als Streckmittel können z,B. auch organische Lösungsmittel als Hilfslösungsmittel verwendet werden. Als flüssige Lösungsmittel kommen im Wesentlichen infrage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol, sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosal-Treibgase, wie Halogenkohlenwasserstoffe sowie Butan, Propan, Stickstoff und Kohlendioxid. Als feste Trägerstoffe kommen in Frage; z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen infrage: z.B. gebrochene und fraktionierte natürliche Gesteine wie Calcit, Bims, Marmor, Sepiolith, Dolomit sowie synthetische Granulate aus anorganischen und organischen Mehlen sowie Granulate aus organischem Material wie Sägemehl, Kokosnussschalen, Maiskolben und Tabakstängel. Als Emulgier und/oder schaumerzeugende Mittel kommen infrage: z.B. nichtionogene und anionische Emulgatoren, wie Polyoxyethylen-Fettsäureester, Polyoxyethylen-Fettalkoholether, z.B. Alkylarylpolyglycolether, Alkylsulfonate, Alkylsulfate, Arylsulfonate sowie Eiweißhydrolysate. Als Dispergiermittel kommen infrage. z.B. Lignin-Sulfitablaugen und Methylcellulose.

Es können in den Formulierungen Haftmittel, wie Carboxymethylcellulose, natürliche und synthetische pulverige, körnige Oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe, wie anorganische Pigmente, z.B. Eisenoxid, Titanoxid, Ferrocyanblau, und organische Farbstoffe, wie Alizarin-, Azo-und Metahphthalacyaninfarbstoffe, und Spurennährstoffe, wie Salze von Eisen, Mangan, Bor, Kupfer, Kobalt Molybdän und Zink, verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Fungiziden, Bakteriziden, Akariziden, Nematiziden oder Insektiziden verwendet werden, um so z.B. das Wirkungsspektrum zu verbreitern oder Resistenzentwicklungen vorzubeugen. In vielen Fällen erhält man dabei synergistische Effekte, d.h. die Wirksamkeit der Mischung ist größer als die Wirksamkeit der Einzelkomponenten.

Als Mischpartner kommen zum Beispiel folgende Verbindungen infrage:

### Fungizide:

1) Inhibitoren der Nukleinsäuresynthese: z.B. Benalaxyl, Benalaxyl-M, Bupirimate, Clozylacon, Dimethirimol, Ethirimol, Furalaxyl, Hymexazol, Mefenoxam, Metalaxyl, Metalaxyl-M, Ofurace, Oxadixyl, Oxolinic acid;
2) Inhibitoren von Mitose und Zellteilung, z.B. Benomyl, Carbendazim, Diethofencarb, Ethaboxam, Fuberidazole, Pencycuron, Thiabendazote, Thiophanate-methyl, Zoxamide;
3) Inhibitoren der Respiration (Atmungsketten-Inhibitoren);
   3,1) Inhibitoren am Komplex I der Atmungskette: z.B. Diflumetorim;
   3.2) Inhibitoren am Komplex II der Atmungskette: z.B. Boscalid, Carboxin, Fenfuram, Flutolanil, Furametpyr, Furmecyclox, Mepronil, Oxycarboxin, Penthiopyrad, Thifluzamide;
   3.3) Inhibitoren am Komplex in der Atmungskette: z,B. Amisulbrom, Azoxystrobin, Cyazofamid, Dimoxystrobin, Enestrobin, Famoxadone, Fenamidone, Fluoxastrobin, Kresoxim-methyl, Metominostrobin, Orysastrobin, Picoxystrobin, Pyraclostrobin, Trifloxystrobin;
4) Entkoppler: z.B. Dinocap, Fluazinam, Meptyldinocap;
5) Inhibitoren der ATP Produktion: z.B. Fentin acetate, Fentin chloride, Fentin hydroxide, Silthiofam;
6) Inhibitoren der Aminosäure- und Protein-Biosynthese; z,B. Andoprim, Blasticidin-S, Cyprodinil, Kasugamycin, Kasugamycin hydrochloride hydrate, Mepanipyrim, Pyrimethanil;
7) Inhibitoren der Signaltransduktion: z.B. Fenpiclonil, Fludioxonil, Quinoxyfen;
8) Inhibitoren der Lipid- und Membran-Synthese: z.B. Biphenyl, Chlozolinate, Edifenphos, Etridiazole, Iodocarb, Iprobenfos, Iprodione, Isoprothiolane, Procymidone, Propamocarb, Propamocarb hydrochloride, Pyrazophos, Tolclofos-methyl, Vinclozolin;
9) Inhibitoren der Ergosterol-Biosynthese: z.B. Aldimorph, Azaconazole, Bi*tert*anol, Bromuconazole, Cyproconazole, Diclobutrazole, Difenoconazole, Diniconazole, Diniconazole-M, Dodemorph, Dodemorph acetate, Epoxiconazole, Etaconazole, Fenarimol, Fenbuconazole, Fenhexamid, Fenpropidin, Fenpropimorph, Fluquinconazole, Flurprimidol, Flusilazole, Flutriafol, Furconazole, Furconazole-cis, Hexaconazole, Imazalil, Imazalil sulfate, Imibenconazole, Ipconazole, Metconazole, Myclobutanil, Naftifine, Nuarimol, Oxpoconazole, Paclobutrazol, Pefurazoate, Penconazole, Prochloraz, Propiconazole, Prothioconazole, Pyributicarb, Pyrifenox, Simeconazole, Spiroxamine, Tebuconazole, Terbinafine, Tetraconazole, Triadimefon, Triadimenol, Tridemorph, Triflumizole, Triforine, Triticonazole, Uniconazole, Viniconazole, Voriconazole;
10) Inhibitoren der Zellwandsynthese; z,B, Benthiavalicarb, Dimethomorph, Flumorph, Iprovalicarb, Mandipropamid, Polyoxins, Polyoxorim, Validamycin A;
11) Inhibitoren der Melanin-Biosynthese: z.B. Carpropamid, Diclocymet, Fenoxanil, Phthalide, Pyroquilon, Tricyclazole;
12) Resistenzinduktoren; z.B. Acibenzolar-S-methyl, Probenazole, Tiadinil;
13) Verbindungen mit Multisite-Aktivität: z.B, Bordeaux Mixture, Captafol, Captan, Chlorothalonil, Copper naphthenate, Copper oxide, Copper oxychloride, Kupferzubereitungen wie z.B. Kupferhydroxid, Kupfersulfat, Dichlofluanid, Dithianon, Dodine, Dodine free base, Ferbam, Fluorofolpet, Folpet, Guazatine, Guazatine acetate, Iminoctadine, Iminoctadine albesilate, Iminoctadine triacetate, Mancopper, Mancozeb, Maneb, Metiram, Metiram Zinc, Oxine-Copper, Propineb, Sulphur und Schweferlzubereitungen wie z.B, Calcium polysulphide, Thiram, Tolylfluanid, Zineb, Ziram;
14) eine Verbindung aus der folgenden Liste: (2E)-2-(2-{[6-(3-Chlor-2-methylphenoxy)-5-fluorpyrimidin-4-yl]oxy}phenyl)-2-(methoxyimino)-N-methylacetamid, (2E)-2-{2-[({[(1E)-1-(3-{[(E)-1-Fluor-2-phenylvinyl]oxy}phenyl)ethyliden]amino}oxy)methyl]phenyl}-2-(methoxyimino)-N-methylacetamid, 1-(4-Chlorphenyl)-2-(1H-1,2,4-triazol-1-yl)cycloheptanol, 1-[(4-Methoxyphenoxy)methyl]-2,2-dimehylpropyl-1H-imidazol-1-carboxylat 2,3,5,6-Tetrachlor-4-(methylsulfonyl)pyridin, 2Butoxy-6-iod-3-propyl-4H-chromen-4-on, 2-Chlor-N-(1, 1,3-trimethyl-2,3-dihydro-1H-inden-4-yl)nicotinamid, 2-Phenylphenol und Salze, 3,4,5-Trichlorpyridin-2,6-dicarbonitril, 3-[5-(4-Chlorphenyl)-2,3-dimeylis-oxazolidin-3-yl]pyridin, 5-Chlor-6-(2,4,6-tritluorphenyl)-N-[(1R)-1,2,2-trimethylpropyl][1,2,4]triazo-lo[1,5-a]pyrimidin-7-amin, 5-Chlor-7-(4-methylpiperidin-1-yl)-6-(2,4,6-trifluorphenyl)[1,2,4]triamio-[1,5-a]pyrimidine, 5-Chlor-N-[(1R)-1,2-dimethylpropyl]-6-(2,4,6-trifluorphenyl)[1,2,4]triazolo[1,5-a]-pyrimidin-7-amin, 8-Hydroxyquinolinsulfat, Benthiazole, Bethoxazin, Capsimycin, Carvone, Chinomethionat, Cufraneb, Cyflufenamid, Cymoxanil, Dazomet, Debacarb, Dichlorophen, Diclomezine, Dicloran, Difenzoquat, Difenzoquat Methylsulphate, Diphenylamine, Ferimzone, Flumetover, Fluopicolide, Fluoroimide, Flusulfamide, Fosetyl-Aluminium, Fosetyl-Calcium, Fosetyl-Natrium, Hexachlorbenzol, Irumamycin, Isotianil, Methasulfocarb, Methyl (2E)-2-{2-[({eyctopropyl[(4-methoxyphenyt)-imino]methyl}thio)methyl]phenyl}-3-methoxyacrylat, Methyl 1-(2,2-dimethyl-2,3-dihydro-1H-inden-1-yl)-1H-imidazol-5-carboxylat, Methyl Isothiocyanate, Metrafenone, Mildiomycin, N-[2-(1,3-Dimethylbutyl)phenyl]-5-fluor-1,3-dimethyl-1H-pyrazol-4-carboxamid, N-(3',4'-Dichlor-5-fluorbiphenyl-2-yl)-3-(difluormethyl)-1-methyl-1H-pyrazol-4-carboxamid (Bixafen), N-(3-Ethyl-3,5,5-trimethylcyclo hexyl)-3-(formylamino)-2-hydroxybenzamide, N-(4-Chlor-2-nitrophenyl)-N-ethyl-4-methylbenzensul-fonamid, N-(4-Chlorbenzyl)-3-[3-methoxy-4-(prop-2-yn-1-yloxy)phenyl]propanamid, N-[(4-Chlorphenyl)(cyano)methyl]-3-[3-methoxy-4-(prop-2-yn-1-yloxy)phenyl]propanamide, N-[(5-Brom-3-chlor-pyridin-2-yl)methyl]-2,4-dichlornicotimamid, N-[1-(5-Brom-3-chlorpyridin-2-yl)ethyl]-2,4-dichlornico-tinamid, N-[l-(5-Brom-3-chlorpyridin-2-yl)ethyl]-2-fluor-4-Iodnicotinamid, N-[2-(4-{[3-(4-Chlorphenyl)prop-2-yn-1-yl]oxy} -3 -methoxyphenyl)ethyl]-N -(rn ethylsulfonyl)yalinamid, N-{(ZH(Cyclopropyl-methoxy)imino][6(difluormethoxy)-2,3-difluorphonyl]methyl}-2-phenylacetamid, N-{2-[1,1'-Bi(cyclopropyl)-2-yl]phenyl}-3-(difluomethyl)-1-meyl-1H-pyrazol-4-carboxamid, N-{2-[3-Chlor-5-(tri-fluomethyl)pyridin-2-yl]ethyl}-2-(triftuormethyl)benzamid (Fluopyram), Natamycin, N- Ethyl-N-methyl-N'{2-methyl-5-(trifluornlethyl)-5-[3-(trimethylsilyl)propoxy]phenyl}imidoformamid, N-Ethyl-N-methyl-N'-{2-methyl-5-(difluonnethyl)-4-[3-(trimethylsilyl)propoxy]phenyl}imidoformamid, Nickeldimethyldithiocarbiunat, Nitrothal-isopropyl, O-{1-[(4-Methoxyphenoxy)methyl]-2,2-dimethylpropyl}-1H-imidazol-1-carbothioat, Octhilinone, Oxamocarb, Oxyfenthiin, Pentachlorophenol und Salze, Phosphorsäure und deren Salze, Piperalin, Propamwub-Fosetylat Propanosine-Natrium, Proquinazid, Pyribencarb, Pyrrolnitrine, Quintozene, 5-Allyl-5-amino-2-isopropyl-4-(2-methylphenyl)-3-oxo-2,3-dihydro-1H-pyrazol-1-carbothioat, Tecloftalam, Tecnazene, Triazoxide, Trichlamide, Valiphenal, Zarilamid,

### Bakterizide:

Bronopol, Dichlorophen, Nitrapyrin, Nickel-Dimethyldithiocarbamat, Kasugamycin, Octhilinon, Furancarbonsäure, Oxytetracyclin, Probenazol, Streptomycin, Tecloftalam, Kupfersulfat und andere KupferZubereitungen.

### Insektizide / Akarizide / Nematizide:

*1. Acetylcholinesterase (AChE) Inhibitoren*
   1.1 Carbamate (z.B. Alanycarb, Aldicarb, Aldoxycarb, Allyxycarb, Aminocarb, Azamethiphos, Bendiocarb, Benfuracarb, Bufencarb, Butacarb, Butocarboxim, Butoxycarboxim, Carbaryl, Carbofuran, Carbosulfan, Chloethocarb, Coumaphos, Cyanofenphos, Cyanophos, Dimetilan, Ethiofencarb, Fenobucarb, Fenothiocarb, Formetanate, Furathiocarb, Isoprocarb, Metam-sodium, Methiocarb, Methomyl, Metolcarb, Oxamyl, Pirimicarb, Promecarb, Propoxur, Thiodicarb, Thiofanox, Triazamate, Trimethacarb, XMC, Xylylcarb)
   1.2 Organophosphate (z.B. Acephate, Azamethiphos, Azinphos (-methyl, -ethyl), Bromophos-ethyl, Bromfenvinfos (-methyl), Butathiofos, Cadusafos, Carbophenothion, Chlorethoxyfos, Chlorfenvinphos, Chlormephos, Chlorpyrifos (-methyl/-ethyl), Coumaphos, Cyanofenphos, Cyanophos, Chlorfenvinphos, Demeton-S-methyl, Demeton-S-methylsulphon, Dialifos, Diazinon, Dichlofenthion, Dichlorvos/DDVP, Dicrotophos, Dimethoate, Dimethylvinphos, Dioxabenzofos, Disulfoton, EPN, Ethion, Ethoprophos, Etrimfos, Famphur, Fenamiphos, Fenitrothion, Fensulfothion, Fenthion, Flupyrazofos, Fonofos, Formothion, Fosmethilan, Fosthiazate, Heptenophos, Iodofenphos, Iprobenfos, Isazofos, Isofenphos, Isopropyl O-salicylate, Isoxathion, Malathion, Mecabam, Methacrifos, Methamidophos, Methidathion, Mevinphos, Monocrotophos, Naled, Omethoate, Oxydemeton-methyl, Parathion (-methyl/-ethyl), Phenthoate, Phorate, Phosalone, Phosmet, Phosphamidon, Phosphocarb, Phoxim, Pirimiphos (-methyl/-ethyl), Profenofos, Propaphos, Propetamphos, Prothiofos, Prothoate, Pyraclofos, Pyridaphenthion, Pyridathion, Quinalphos, Sebufos, Sulfotep, Sulprofos, Tebupirimfos, Temephos, Terbufos, Tetrachlorvinphos, Thiometon, Triazophos, Triclorfon, Vamidothion)
*2. Natrium-Kanal-Modulatoren* / *Spannungsabhängige Natrium-Kanal-Blocker*
   2.1 Pyrethroide (z.B. Acrinathrin, Allethrin (d-cis-trans, d-trans), Beta-Cyfluthrin, Bifenthrin, Bioallethrin, Bioallethrin-S-cyclopentyl-isomer, Bioethanomethrin, Biopermethrin, Bioresmethrin, Chlovaporthrin, Cis-Cyperinebin, Cis-Resmethriti, Cis-Permethrin, Clocythrin, Cycloprothrin, Cyfluthrin, Cyhalothrin, Cypermethrin (alpha-, beta-, theta-, zeta-), Cyphenothrin, DDT, Deltamethrin, Empenthrin (IR-isomer), Esfenvalerate, Etofenprox, Fenfluthrin, Fenpropathrin, Fenpyrithrin, Fenvalerate, Flubrocythrinate, Flucythrinate, Flufenprox, Flumethrin, Fluvalinate, Fubfenprox, Gamma-Cyhalothrin, Imiprothrin, Kadethrin, Lambda-Cybalothrin, Metofluthrin, Permethrin (cis-, trans-), Phenothrin (1R-trans isomer), Prallethrin, Profluthrin, Protrifenbute, Pyresmethrin, Resmethrin, RU 15525, Silafluofen, Tau-Fluvalinate, Tefluthrin, Terallethrin, Tetramethrin (1R-isomer), Tralomethrin, Transfluthrin, ZXI 8941, Pyrethrins (pyrethrum))
   2.2 Oxadiazine (z.B. Indoxacarb)
*3. Acetylcholin-Rezeptor-Agontstenl-AntagonJsten*
   3.1 Chloronicotinyle/Neonicotinoide (z,B, Acetamiprid, Clothianidin, Dinotefuran, Imidacloprid, Nitenpyram, Nithiazine, Thiacloprid, Thiamethoxam)
   3.2 Nicotine, Bensultap, Cartap
*4. Acetylcholin-Rezeptor-Modulatoren*
   4.1 Spinosyne (z.B. Spinosad)
*5. GABA-gesteuerte Chlorid-kanal-Antagonisten*
   5.1 Cyclodiene Organochloxine (z.B. Camphechlor, Chlordane, Endosulfan, Gamma-HCH, HCH, Heptachlor, Lindane, Methoxychlor
   5.2 Fiprole (z.B. Acetoprole, Ethiprote, Fipronil, Vaniliprole)
*6. Chlorid-Kanal-Aktivatoren*
   6.1 Mectine (z.B. Abamectin, Avermectin, Emamectin, Emamectin-benzoate, Ivermectin, Milbemectin, Milbemycin)
*7. Juventlhormon-Mimetika:*
   (z.B. Diofenolan, Epofenonane, Fenoxycarb, Hydroprene, Kinoprene, Methoprene, Pyriproxifen, Triprene)
*8, Ecdysonagonisten*/*disruptoren*
   8.1 Diacylhydrazine (z.B. Chromafenozide, Halofenozide, Methoxyfenozide, Tebufenozide)
*9. Inhibitoren der Chitinbiosynthese*
   9.1 Benzoylhatnstoffe (z.B. Biestrifluron, Chlofluazuron, Diflubenzuron, Fluazuron, Flucycloxuron, Flufenoxuron, Hexaflumuron, Lufenuron, Novaluron, Noviflumuron, Penfluron, Teflubenzuron, Triflumuron)
   9.2 Buprofezin
   9.3 Cyromazine
*10. Inhibitoren der oxidativen Phosphorylierung, ATP-Disruptoren*
   10.1 Diafenthiuron
   10.2 Organotine (z.B. Azocyclotin, Cyhexatin, Fenbutatin-oxide)
*11. Entkoppler der oxidativen Phoshorylierung durch Unterbrechung des H-Protongradienten*
   11.1 Pyrrole (z.B. Chlorfenapyr)
   11.2 Dinitrophenole (z.B. Binapacyrl, Dinobuton, Dinocap, DNOC)
*12. Seite-I-Elektronentramportinhibitoren*
   12.1 METTs (z.B. Fenazaquin, Fenpyroximate, Pyrimidifen, Pyridaben, Tebufenpyrad, Tolfenpyrad)
   12.2 Hydramethylnone
   12,3 Dicofol
*13. Seite-II-Elektronentranspartinhibitoren*
   13.1 Rotenone
*14. Seite-III-Elektronentransportinhibitoren*
   14.1 Acequinocyl, Fluacrypyrim
*15, Mikrobielle Disruptoren der Insektendarmmembran*
   Bacillus thuringiensis-Stämme
*16, Inhibitoren der Fettsynthese*
   16.1 Tetronsäuren (z.B. Spirodiclofen, Spiromesifen)
   16,2 Tetramsäuren [z.B. 3-(2,5-Dimethylphenyl)-8-methoxy-2-oxo-1-azaspiro[4,5]dec-3-en-d-yl ethyl carbonate (alias: Carbonic acid, 3-(2,5-dimethylphenyl)-8-methoxy-2-oxo-1-azaspiro[4,5]dec-3-en-4-yl ethyl ester, CAS-Reg.-No,: 382608-10-8) and Carbonic acid, cis-3-(2,5-dimethylphenyl)-8-methoxy-2-oxo-1-azaspiro[4.5]dec-3-en-4-yl ethyl ester (CAS-Reg.-No.: 203313-25-1)]
*17. Carboxamide*
   (z.B. Flonicamid)
*18. Oktopaminerge Agonisten*
   (z.B. Amitraz)
*19. Inhibitoren der Magnesium-stimulierten ATPase*
   (z.B. Propargite)
*20. Agonisten des Ryanodin-Rezeptors,*
   20.1 Benzoesäuredicarboxamide [z.B. N²-[1,1-Dimethyl-2-(methylsulfonyl)ethyl]-3-iod-N¹-[2-methyl-4-[1,2,2,2-tetrafluor-1-(trifluormethyl)ethyl]phenyl]-1,2-banxenedicarboxamide (CAS-Reg.-No.: 272451-65-7), Flubendiamide]
   20.2 Anthranilamide (z.B. DPX E2Y45 = 3.Brom-N-{4-chlor-2-methyl-6-[(methylamino)carbonyl]-phenyl} -1-(3-chlorpyridin-2-yl)-1H-pyrazol-5-carboxamid)
*21. Nereistoxin-Analoge*
   (z.B. Thiocyclam hydrogen oxalate, Thiosultap-sodium)
*22. Biologika, Hormone oder Pheromone*
   (z.B. Azadirachtin, Bacillus spec., Beauveria spec., Codlemone, Metarrhizium spec., Paecilomyces spec., Thuringiensin, Verticillium spec.)
*23. Wirkstoffe mit unbekannten oder nicht spezifischen Wirkmechmismen*
   23.1 Begesungsmittel (z.B. Aluminium phosphide, Methyl bromide, Sulfuryl fluoride)
   23.2 Selektive Fraßhemmer (z.B. Cryolite, Flonicamid, Pymetrozine)
   23.3 Milbenwachstumsinhibitoren (z.B. Clofentezine, Etoxazole, Hexythiazox)
   23.4 Amidoflumet, Benclothiaz, Benzoximate, Bifenazate, Bromopropylate, Buprofezin, Chinomethionat, Chlordimeform, Chlorobenzilate, Chloropicrin, Clothiazoben, Cycloprene, Cyflumetofen, Dicyclanil, Fenoxacrim, Fentrifanil, Flubenzimine, Flufenerim, Flutenzin, Gossyplure, Hydramethylnone, Japonilure, Metoxadiazone, Petroleum, Piperonyl butoxide, Potassium oleate, Pyrafluprole, Pyridalyl, Pyriprole, Sulfluramid, Tetradifon, Tetrasul, Triarathene, Verbutin, ferner die Verbindung 3-Methylphenyl-propylcubamat (Tsumacide Z), die Verbindung 3-(S-Ch!or-3-pyridmyt).8-(2,2,2-triHuorethyl)-8-azabicyclo[3.2.1]o,r,tan-3-carbonitrü (CAS-Reg: Nr. 185982-80-3) und das entsprechende 3-endo-Isomere (CAS-Reg.-Nr. 185984-60-5) (vgl. WO 96/37494, WO 98/25923), sowie Präparate, welche insektizid wirksame Pflanzenextrakte, Nematoden, Pilze oder Viren enthalten.

Auch eine Mischung mit anderen bekannten Wirkstoffen, wie Herbiziden, oder mit Düngemitteln und Wachstumsregulatoren, Safenern bzw, Semiochemicals ist möglich.

Darüber hinaus weisen die erfindungsgemäßen Verbindungen der Formel (I) auch sehr gute antimykotische Wirkungen auf. Sie besitzen ein sehr breites antimykotisches Wirkungsspektrum, insbesondere gegen Dermatophyten und Sprosspilze, Schimmel und diphasische Pilze (z,B. gegen Candida-Spezies wie Candida albicans, Candida glabrata) sowie Epidormophyton floccosum, Aspergillus-Spezies wie Aspergillus niger und Aspergillus fumigatus, Trichophyten-Spezies wie Trichophyton mentagrophytes, Microsporon-Spezies wie Microsporon canis und audouinii. Die Aufzählung dieser Pilze stellt keinesfalls eine Beschränkung des erfassbaren mykotischen Spektrums dar, sondern hat nur erläuternden Charakter.

Die Wirkstoffe können als solche, in Form ihrer Formulierungen oder den daraus bereiteten Anwendungsformen, wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt worden.

Beim Einsatz der erfindungsgemäßen Wirkstoffe als Fungizide können die Aufwandmengen je nach Applikationsart innerhalb eines größeren Bereiches variiert werden. Bei der Behandlung von Pflanzenteilen liegen die Aufwandmengen an Wirkstoff im allgemeinen zwischen 0,1 und 10.000 g/ha, vorzugsweise zwischen 10 und 1,000 g/ha. Bei der Saatgutbehandlung liegen die Aufwandmengen an Wirkstoff im allgemeinen zwischen 0,001 und 50 g pro Kilogramm Saatgut, vorzugsweise zwischen 0,01 und 10 g pro Kilogramm Saatgut. Bei der Behandlung des Bodens liegen die Aufwandmengen an Wirkstoff im allgemeinen zwischen 0,1 und 10.000 g/ha, vorzugsweise zwischen 1 und 5.000 g/ha.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Der Begriff "Teile" bzw. "Teile von Pflanzen" oder "Pflanzenteile" wurde oben erläutert.

Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Technikon gezüchtet worden sind. Dies können Sorten, Rassen, Bio-und Genotypen sein.

Je nach Pflanzenarten bzw. Pflanzensorten, deren Standort und Wachstumsbedingungen (Böden, Klima, Vegatationsperiode, Ernährung) können durch die erfindungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel, besseres Pflanzenwachstum, erhöhte Toleranz gegenüber hohen oder niedrigen Temperaturen, erhöhte Toleranz gegen Trockenheit oder gegen Wasser-bzw. Bodonsalzgehalt, erhöhte Blühleistung, erleich*tert*e Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte möglich, die Über die eigentlich zu erwartenden Effekte hinausgehen.

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser-bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe, Als Beispiel transgener Pflanzen werden die wichtigen Kulturpflanzen, wie Getreide (Weizen, Reis), M4is, Soja, Kartoffel, Baumwolle, Tabak, Raps sowie Obstpflanzen (mit den Früchten Äpfel, Birnen, Zitrusfrüchten und Weintrauben) erwähnt, wobei Mais, Soja, Kartoffel, Baumwolle, Tabak und Raps besonders hervorgehoben werden. Als Eigenschaften ("Traits") werden besondere hervorgehoben die erhöhte Abwehr der Pflanzen gegen Insekten, Spinnentiere, Nematoden und Schnecken durch in den Pflanzen entstehende Toxine, insbesondere solche, die durch das genetische Material aus Bacillus Thuringiensis (z.B. durch die Gene CryIA(a), CryLA(b), CryIA(p), CryIIA, CrymA, CryIIB2, Cry9c Cry2Ab, Cry3Bb und CryIF sowie deren Kombinationen) in den Pflanzen erzeugt werden (im folgenden "Bt Pflanzen"). Als Eigenschaften ("Traits") werden auch besonders hervorgehoben die erhöhte Abwehr von Pflanzen gegen Pilze, Bakterien und Viren durch Systemische Akquirierte Resistenz (SAR), Systemin, Phytoalexine, Elicitoren sowie Resistenzgene und entsprechend exprimierte Proteine und Toxine. Als Eigenschaften ("Traits") werden weiterhin besonders hervorgehoben die erhöhte Toleranz der Pflanzen gegenüber bestimmten herbiziden Wirkstoffen, beispielsweise Imidazolinonen, Sulfonylharnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeichnungen YIELD GARD® (z.B. Mais, Baumwolle, Soja), KnockOut® (z.B. Mais), StarLink® (z.B. Mais), Bollgard® (Baumwolle), Nucoton® (Baumwolle) und NewLeaf® (Kartoffel) vertrieben werden. Als Beispiele für Herbizid tolerante Pflanzen seien Maissorten, Baumwollsorten und Sojasorten genannt, die unter den Handelsbezeichnungen Roundup Ready® (Toleranz gegen Glyphosate z.B. Mais, Baumwolle, Soja), Liberty Link® (Toleranz gegen Phosphinotricin, z.B. Raps), IMI® (Toleranz gegen Imidazolinone) und STS® (Toleranz gegen Sulfonylharnstoffe z.B. Mais) vertrieben werden. Als Herbizid resistente (konventionell auf Herbizid-Toleranz gezüchtete) Pflanzen seien auch die unter der Bezeichnung Clearfield® vertriebenen Sorten (z.B. Mais) erwähnt. Selbstverständlich gelten diese Aussagen auch für in der Zukunft entwickelte bzw. zukünftig auf den Markt kommende Pflanzensorten; mit diesen oder zukünftig entwickelten genetischen Eigenschaften ("Traits").

Die aufgeführten Pflanzen können besonders vorteilhaft erfindungsgemäß mit den Verbindungen der allgemeinen Formel (I) bzw. den erfindungsgemäßen Wirkstoffmischungen behandelt werden. Die bei den Wirkstoffen bzw. Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mischungen.

Die Herstellung (siehe Schema 5) und die Verwendung der erfindungsgemäßen Wirkstoffe geht aus den folgenden Beispielen hervor.

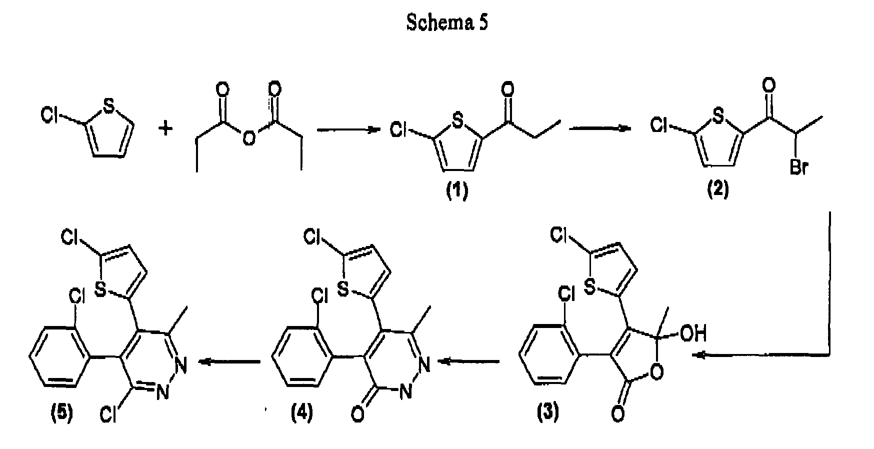

### 1-(5-Chlor-2-thienyl)propan-1-on (1) (analog US4024156)

42 g (354 mmol, 1 eq.) 2-Chlorthiophen und 46.8 mL (365 mmol, 1.03 eq.) Propionsäureanhydrid werden bei RT vorgelegt und bei gegebener Temperatur 5.43 g 38 mmol, 0.108 eq.) Bortrifluorid-Diethyletherkomplex in einer Portion zugegeben; dabei steigt die Temperatur langsam auf 50°C. Das Gemisch wird eine Stunde nachgerührt; dabei sinkt die Temperatur langsam auf 30°C. Anschliessend wird 30 min. auf 90°C erhitzt, dann abgekühlt und das Reaktionsgemisch auf Eiswasser gegossen. Die wässrige Phase wird mehrfach mit Methylenchlorid extrahiert, getrocknet und einrotiert, Nach Destillation des Rohprodukts (0.5 mbar, 54°C) wurden 37.5 g (60% Ausbeute) 1-(5-Chlor-2-thienyl)propan-1-on als farbloser Feststoff erhalten.

### 2-Brom-1-(5-chlor-2-thienyl)propan-1-on (2) (analog J. Am. Chem. Soc. 1950, 72, 3659)

6 g (34.4 mmol, 1 eq.) 1-(5+Chlor-2-thienyl)propan-1-on werden in 68 mL Methylenchlorid gelöst, portionsweise mit 12.2 g (34,4 mmol, 1eq.) 90%igem Pyridiniumbromidperbromid versetzt und 16 Stunden bei Raumtemperatur gerührt. Zur Aufarbeitung wird das Reaktionsgemisch mit IN HCl verrührt, die organische Phase abgetrennt, getrocknet und einrotiert. Es wurden 8.4 g (94% Ausbeute, 96%ig) 2-Brom-1-(5-chlor-2-thienyl)propan-1-on als braunes Öl erhalten.

### 3-(2-Chlorphenyl)-4-(5-chlor-2-thienyl)-5-hydroxy-5-methylfuran-2(5H)-on (3)

1.48 g (8.7 mmol, 1.1eq.) 2-Chlorphenylessigsäure werden in 30 ml Acetonitril vorgelegt. Dazu tropft man eine Mischung von 1.21 mL (8.7 mmol,1.1 eq.) Triethylamin und 10 m1 Acetonitril und rührt 20min. bei Raumtemperatur nach. Anschließend gibt man eine Lösung von 2 g (7.89 mmol, 1 eq.) 2-Brom-1-(5-chlor-2-thienyl)propan-1-on in 10 ml Acetonitril tropfenweise zu und rührt die Reaktionsmischung 16 Stunden bei gegebener Temperatur. Das Reaktionsgemisch wird nun auf 0°C gekühlt und tropfenweise mit einer Lösung von 3.53 mL (23.66 mmol, 3 eq.) Diazabicyclo(S .4,O)undecan in 50 mL Acetonitril versetzt. Anschliessend wird 2 Stunden bei gegebener Temperatur nachgerührt und das Gemisch langsam auf RT erwärmt. Nun wird für einen Zeitraum von 3 Stunden Luft durch das Reaktionsgemisch geleitet. Nach beendeter Reaktion wird das Gemisch mit 1N HCl und Methylenchlorid verrührt, die organische Phase abgetrennt, getrocknet und einrotiert. Nach chromatographischer Reinigung an Silicagel wurden 1.3 g (36% Ausbeute, 74%ig, logP = 3.91) 3-(2-Chlorphenyl)-4-(5-chlor-2-thienyl)-5-hydroxy-5-methylfuran-2(5H)-on als farbloses Öl erhalten.

Analog können beispielsweise folgende Verbindungen der Formel (VII) hergestellt werden:
3 -(2-Chlor-6- fluorphenyl)-4-(5-chlor-2-thienyl)-5-hydroxy-5-methylfuran-2(5H)-on (farbloses Öl, logP = 3.13)
4-(5-Chlor-2-thienyl)-3-(2,6-difluorphonyl)-5-hydroxy-5-methylfuran-2(5H)-on (farbloses Öl, logP = 2.94)
4-(2,6-Difluorphenyl)-3-(5-Methyl-2-furyl)-5-hydroxy-5-methylfuran-2(5H)-on (farbloses Öl, logP = 2.35)
3-(2,6-Difluorphenyl)4-(2-thienyl)-5-hydroxy-5-methylfuran-2(5H)-on (farbloses Öl, logP = 2.39)
3-(2,6-Difluorphonyl)-4-(2-thiazolyl)-5-hydroxy-5-methylfuran-2(5H)-on (farbloses Öl, logP = 1.92)
3-(2,6-Difluorphenyl)-4-(5,6-Dimethyl-2-pyridyl)-5-hydroxy-5-methylfuran-2(5H)-on (farbloses Öl, logP = 2.65)
3-(2,6-Dichlorphenyl)-4-(5,6-Dimethyl-2-pyridyl)5-hydroxy-5-methylfuran-2(5H)-on (farbloses Öl, logP = 3.14)
3-(2,4,5-Trilluorphenyl)4-(5,6-Dimethyl-2-pyridyl)-5-hydroxy-5-methylfuran-2(5H)-on (farbloses Öl, logP = 2.89)
3-(2,6-Difluorphenyl)-4-(4-pyridyl)-5-hydroxy-5-methylfuran-2(5H)-on (farbloses Öl, logP = 1.4)
3-(2-Methyl)-4-(2-thienyl)-5-hydroxy-5-methylfuran-2(5H)-on (farbloses Öl, logP = 2.59)
3-(2-Chlor-4-fluorphenyl)-4-(5-chlor-2-thienyl)-5-hydroxy-5-methylfuran-2(5H)-on (farbloses Öl, logP = 3.24)
3-(3-Chlorphenyl)-4-(5-chlor-2-thienyl)-5-hydroxy-5-metylfuran-2(5H)-on (farbloses Öl, logP = 3.38)
4-(5-Chlor-2-thienyl)-3-(2,6-dichlorphenyl)-5-hydroxy-5-methylfuran-2(5H)-on (farbloses Öl, logP = 3.26)
4-(5-Chlor-2-thienyl)-3-(2-fluorphenyl)-5-hydroxy-5-methylfuran-2(5H)-on (farbloses Öl, logP = 2.93)
3-(2-Brom-4-fluorphenyl)-4-(5-chlor-2-thienyl)-5-hydroxy-5-methylfuran-2(5H)-on (farbloses Öl, logP= 3.30)
3-(2-Fluorphenyl)-4-(2-thienyl)-5-hydroxy-5-methylfuran-2(5H)-on (farbloses Öl, logP = 2.29)
3-(2-Chlorphenyl)-4-(2-thienyl)-5-hydroxy-5-methylfuran-2(5H)-on (farbloses Öl, logP = 2.44)
4-(5-Chlor-2-thienyl)-3-(2,2-difluor-1,3-benzodioxol-4-yl)-5-hydroxy-5-methylfuran-2(5H)-on (farbloses Öl, logP = 3.41)
4.(5-Chlor-2-thienyl)-3-(2,4,5-trifluorphenyl)-5-hydroxy-5-methylfuran-2(5H)-on (farbloses Öl, logP = 3.17)
3-(2-Chlor-6-fluorphenyl)-4-(2-thienyl)-5-hydroxy-5-methylfuran-2(5H)-on (farbloses Öl, logP = 2.61)
3-(2-Chlor-4-fluorphenyl)-4-(2-thienyl)-5-hydroxy-5-methylfuran-2(5H)-on (farbloses Öl, logP = 2.68)
3-(3-Chlorphenyl)-4-(2-thienyl)-5-hydroxy-5-methylfuran-2(5H)-on (farbloses Öl, logP = 2.73)
3-(2,4,5-Trifluorphonyl)-4-(2-thienyl)-5-hydroxy-5-methylfuran-2(5H)-on (farbloses Öl, logP = 2.57)
3-(2-Brom-4-fluorphenyl)-4-(2-thienyl)-5-hydroxy-5-methylfuran-2(5H)-on (farbloses Öl, logP = 2.64)
3-(2,6-Dichlorphenyl)-4-(2-thienyl)-5-hydroxy-5-methylfuran-2(5H)-on (farbloses Öl, logP= 2.57)
**4-(2-Chlorphenyl)-5-(5-chlor-2-thienyl)-6-methylpyridazin-3(2H)-on (4)**

1.3 g (2,82 mmol, 1 eq.) 74 %iges 3-(2-Chlorphenyl)-4-(5-chlor-2-thienyl)-5-hydroxy-5-methylfuran-2(5H)-on werden in 20 mL n-Butanol gelöst, tropfenweise mit 0.27 mL (5.6 mmol, 2 eq.) Hydrazinhydrat versetzt und 16 Stunden bei 100°C gerührt Die Hälfte des Lösungsmittels wird am Rotationsverdampfer entfernt und das Gemisch im Eisbad auf 0°C gekühlt. Nach einer Stunde wird der kristalline Feststoff abgesaugt und getrocknet. Es wurden 0.75 g (72% Ausbeute, 91%ig; logP = 2,83) 4-(2-Chlorphenyl)-5-(5-chlor-2-thienyl)-6-methylpyridazin-3(2H)-on als farbloser Feststoff erhalten.

Analog können beispielsweise folgende Verbindungen der Formel (VIII) hergestellt werden;
4-(2-Chlor-4-fluorphenyl)-5-(5-chlor-2-thienyl)-6-methylpyridazin-3(2H)-on (farbloser Feststoff; logP = 2.97)
4-(3-Chlorphenyl)-5-(5-chlor-2-thienyl)-6-methylpyridazin-3(2H)-on (farbloser Feststoff; logP = 3.04)
5-(5-Chlor-2-thienyl)-4-(2,6-dichlorphenyl)- 6-methylpyridazin-3(2H)-on (farbloser Feststoff; logP =3.11)
5-(5-Chlor-2-thienyl)-4-(2-fluorphenyl)- 6-methylpyridazin-3(2H)-on (farbloser Feststoff; logP = 2.64)
4-(2-Brom-4-fluorphenyl)-5-(5-chlor-2-thienyl)-6-methylpyridazin-3(2H)-on (farbloser Feststoff; logP = 3.03)
4-(2-Brom-4-fluorphenyl)-5-(2-thienyl)-6-methylpyridizin-3(2H)-on (farbloser Feststoff; logP = 2.3 8)
4-(2-Fluorphonyl)-5-(2-thienyl)-6-methylpyridazin-3(2H)-on (farbloser Feststoff; logP = 2.06)
4-(2-Chlorphenyl)-5-(2-thienyl)-6-methylpyridazin-3(2H)-on (farbloser Feststoff; logP = 2.19)
4-(2,6-Dichlorphenyl)-5-(2-thienyl)-6-methylpyridazin-3(2H)-on (farbloser Feststoff-, logP = 2.43)
5-(5-Chlor-2-thienyl)-4-(2,2-difluor-1,3-benzodioxol-4-yl)-6-methylpyridazin-3(2H)-on (farbloser Feststoff; logP= 3.18)
5-(5-Chlor-2-thienyl)-4-(2,4,5-trifluorphanyl)- 6-methylpyridazin-3(2H)-on (farbloser Feststoff; logP = 2,88)
5-(2-thienyl)-4-(2,4,5-trifluorphenyl)- 6-methylpyridazin-3(2H)-on (farbloser Feststoff; logP = 2.32)
5-(4-Pyridyl)4-(2,6-difluorphenyl)- 6-methylpyridazin-3(2H)-on (farbloser Feststoff; logP = 0.93)
5-(5-Methyl-2-furyl)-4-(2,6-difluorphenyl)-6-methylpyridazin-3(2H)-on (farbloser Feststoff; logP =2.19)
5-(2-Thiazolyl)-4-(2,6-difluorphenyl)- 6-methylpyridazin-3(2H)-on (farbloser Feststoff; logP = 1.57)
5-(5,6-Dimethyl-2-pyridyl)-4-(2,6-dichlorphenyl)-6-methylpyridazin-3(2H)-on (farbloser Feststoff; logP= 2.11)
5-(5,6-Dimethyl-2-pyridyl)-4-(2,4,5-trifluorphenyl)-6-methylpyridazin-3(2H)-on (farbloser Feststoff; logP = 2.06)

### 3-Chlor-4-(2-Chlorphenyl)-5-(5-chlor-2-thienyl-6-methylpyridazin (5); Beispiel 58

0.75 g (2 mmol,1 eq.) 91%iges 4-(2-Chlorphenyl)-5-(5-chlor-2-thienyl)-6-methylpyridazin-3(2H)-on werden in 3 mL Phosphoroxychlorid eine Stunde bei 110°C gerührt. Anschliessend wird das Gemisch zur Trockne einrotiert, mit Wasser und Methylenchlorid verrührt, die organische Phase abgetrennt, getrocknet und einrotiert. Es wurden 0.6 g (76% Ausbeute, 90%ig; logP = 4,01) 3-Chlor-4-(2-Chlorphenyl)-5-(5-chlor-2-thienyl)-6-methylpyridazin als beigefarbener Feststoff erhalten.

Analog können beispielsweise folgende Verbindungen der Formel (I) hergestellt werden (siehe Tabelle I):

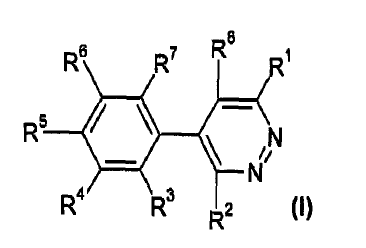

**Tabelle 1**

| **Bsp. Nr.** | **R¹** | **R²** | **R³** | **R⁴** | **R⁵** | **R⁶** | **R**⁷ | **R⁸ (mit R⁹)** | **LogP^{a)}** |
|---|---|---|---|---|---|---|---|---|---|
| 1 | Me | Cl | (Difluormethylen)bis(oxy) | | H | H | H | 5-Chlor-2-thienyl | 4,33 |
| 2 | Me | Cl | (Difluormethylen)bis(oxy) | | H | H | H | 2-Thienyl | |
| 3 | Me | Cl | (Difluormethylen)bis(oxy) | | H | H | H | 5,6-Dimethylpyridin-2-yl | |
| 4 | Me | Cl | (Difluormethylen)bis(oxy) | | H | H | H | Pyridin-4-yl | |
| 5 | Me | Cl | (Difluormethylen)bis(oxy) | | H | H | H | Pyridin-2-yl | |
| 6 | Me | Cl | (Diflluormethylen)bis(oxy) | | H | H | H | Pyridin-3-yl | |
| 7 | Me | Cl | (Difluormethylen)bis(oxy) | | H | H | H | 5-Methyl-2-furyl | |
| 8 | Me | Cl | (Difluormethylen)bis(oxy) | | H | H | H | 1,3-Thiaxol-2-yl | |
| 9 | Me | Cl | (Difluormethylen)bis(oxy) | | H | H | H | Pyrimidin-2-yl | |
| 10 | Me | Cl | Br | H | F | H | H | 5-Chlor-2-thienyl | 4,28 |
| 11 | Me | Cl | Br | H | F | H | H | 2-Thienyl | 3,56 |
| 12 | Me | Cl | Br | H | F | H | H | 5,6-Dimethylpyridin-2-yl | |
| 13 | Me | Cl | Br | H | F | H | H | Pyridin-4-yl | |
| 14 | Me | Cl | Br | H | F | H | H | pyridin-2-yl | |
| 15 | Me | Cl | Br | H | F | H | H | Pyridin-3-yl | |
| 16 | Me | Cl | Br | H | F | H | H | 5-Methyl-2-furyl | |
| 17 | Me | Cl | Br | H | F | H | H | 1,3-Thiazol-2-yl | |
| 18 | Me | Cl | Br | H | F | H | H | Pyrimidin-2-yl | |
| 19 | Me | Cl | Cl | H | Cl | H | Cl | 2-Thienyl | |
| 20 | Me | Cl | Cl | H | Cl | H | Cl | 5-Chlor-2-thienyl | |
| 21 | Me | Cl | Cl | H | Cl | H | Cl | 5,6-Dimethylpyridin-2-yl | |
| 22 | Me | Cl | Cl | H | Cl | H | Cl | Pyridin-4-yl | |
| 23 | Me | Cl | Cl | H | Cl | H | Cl | Pyridin-2-yl | |
| 24 | Me | Cl | Cl | H | Cl | H | Cl | Pyridin-3-yl | |
| 25 | Me | Cl | Cl | H | Cl | H | Cl | 5-Methyl-2-fwyl | |
| 26 | Me | Cl | Cl | H | Cl | H | Cl | 1,3-Thiazol-2-yl | |
| 27 | Me | Cl | Cl | H | Cl | H | Cl | Pyrimidin-2.yl | |
| 28 | Me | Cl | Cl | H | Cl | H | H | 2-Thienyl | |
| 29 | Me | Cl | Cl | H | Cl | H | H | 5-Chlor-2-thienyl | |
| 30 | Me | Cl | Cl | H | Cl | H | H | 5,6-Dimethylpyridin-2-yl | |
| 31 | Me | Cl | Cl | H | Cl | H | H | Pyridin-4-yl | |
| 32 | Me | Cl | Cl | H | Cl | H | H | Pyridin-2-yl | |
| 33 | Me | Cl | Cl | H | Cl | H | H | Pyridin-3-yl | |
| 34 | Me | Cl | Cl | H | Cl | H | H | 5-Methyl-2-furyl | |
| 35 | Me | Cl | Cl | H | Cl | H | H | 1,3-Thiazol-2-yl | |
| 36 | Me | Cl | Cl | H | Cl | H | H | Pyrimidin-2-yl | |
| 37 | Me | Cl | Cl | H | F | H | H | 2-Thienyl | 3,45 |
| 38 | Me | Cl | Cl | H | F | H | H | 5-Chlor-2-thienyl | 4,21 |
| 39 | Me | Cl | Cl | H | F | H | H | 5,6-Dimethylpyridin-2-yl | |
| 40 | Me | Cl | Cl | H | F | H | H | Pyridin-4-yl | |
| 41 | Me | Cl | Cl | H | F | H | H | Pyridin-2-yl | |
| 42 | Me | Cl | Cl | H | F | H | H | Pyridin-3-yl | |
| 43 | Me | Cl | Cl | H | F | H | H | 5.Mathyl-2-furyl | |
| 44 | Me | Cl | Cl | H | F | H | H | 1,3-Thiazol-2-yl | |
| 45 | Me | Cl | Cl | H | F | H | H | Pyrimidin-2-yl | |
| 46 | Me | Cl | Cl | H | H | H | Cl | 5-Chlor-2-thienyl | 4,44 |
| 47 | Me | Cl | Cl | H | H | H | Cl | 5,6-Dimethylpyridin-2-yl | 4,16 |
| 48 | Me | Cl | Cl | H | H | H | Cl | 2-Thienyl | |
| 49 | Me | Cl | Cl | H | H | H | Cl | Pyridin-4-yl | |
| 50 | Me | Cl | Cl | H | H | H | Cl | Pyridin-2-yl | |
| 51 | Me | Cl | Cl | H | H | H | Cl | Pyridin-3-yl | |
| 52 | Me | Cl | Cl | H | H | H | Cl | 5-Methyl-2-furyl | |
| 53 | Me | Cl | Cl | H | H | H | Cl | 1,3-Thiazol-2-yl | |
| 54 | Me | Cl | Cl | H | H | H | Cl | Pyrimidin-2-yl | |
| 55 | Me | Cl | F | H | H | H | Cl | 2-Thienyl | 3,33 |
| 56 | Me | Cl | F | H | H | H | Cl | Pyridin-4-yl | |
| 57 | Me | Cl | F | H | H | H | Cl | Pyridin-3-yl | |
| 58 | Me | Cl | Cl | H | H | H | H | 5.Chlor-2-thienyl | 4,01 |
| 59 | Me | Cl | Cl | H | H | H | H | 2-Thienyl | 3,29 |
| 60 | Me | Cl | Cl | H | H | H | H | 5,6-Dimethylpyridin- | |
| 61 | Me | Cl | Cl | H | H | H | H | Pyridin-4-yl | |
| 62 | Me | Cl | Cl | H | H | H | H | Pyridin-2-yl | |
| 63 | Me | Cl | Cl | H | H | H | H | Pyridin-3-yl | |
| 64 | Me | Cl | Cl | H | H | H | H | 5-Methyl-2-furyl | |
| 65 | Me | Cl | Cl | H | H | H | H | 1,3-Thiazol-2-yl | |
| 66 | Me | Cl | Cl | H | H | H | H | Pyrimidin-2-yl | |
| 67 | Me | Cl | F | H | F | H | F | 5-Chlor-2-thienyl | 4,11 |
| 68 | Me | Cl | F | H | F | H | F | 2-Thienyl | |
| 69 | Me | Cl | F | H | F | H | F | 5,6-Dimethylpyridin-2-yl | |
| 70 | Me | Cl | F | H | F | H | F | Pyridin-4-yl | |
| 71 | Me | Cl | F | H | F | H | F | Pyridin-2-yl | |
| 72 | Me | Cl | F | H | F | H | F | Pyridin-3-yl | |
| 73 | Me | Cl | F | H | F | H | F | 5-Methyl-2-furyl | |
| 74 | Me | Cl | F | H | F | H | F | 1,3-Thiazol-2-yl | |
| 75 | Me | Cl | F | H | F | H | F | Pyrimidin-2-yl | |
| 76 | Me | Cl | F | H | F | H | H | 2-Thienyl | |
| 77 | Me | Cl | F | H | F | H | H | 5-Chlor-2-thienyl | |
| 78 | Me | Cl | F | H | F | H | H | 5,6-Dimethylpyridin-2-yl | |
| 79 | Me | Cl | F | H | F | H | H | Fyridin-4-yl | |
| 80 | Me | Cl | F | H | F | H | H | Pyridin-2-yl | |
| 81 | Me | Cl | F | H | F | H | H | Pyridin-3-yl | |
| 82 | Me | Cl | F | H | F | H | H | 5-Methyl-2-furyl | |
| 83 | Me | Cl | F | H | F | H | H | 1,3-Thiazol-2-yl | |
| 84 | Me | Cl | F | H | F | H | H | Pyrimidin-2-yl | |
| 85 | Me | Cl | F | H | F | F | H | 2-Thienyl | 3,39 |
| 86 | Me | Cl | F | H | F | F | H | 5-Chlor-2-thienyl | 4,01 |
| 87 | Me | Cl | F | H | F | F | H | 5,6-Dimethylpyridin- 2-yl | 3,15 |
| 88 | Me | Cl | F | H | F | F | H | Pyridin-4-yl | |
| 89 | Me | Cl | F | H | F | F | H | Pyridin-2-yl | |
| 90 | Me | Cl | F | H | F | F | H | Pyridin-3-yl | |
| 91 | Me | Cl | F | H | F | F | H | 5-Methyl-2-furyl | |
| 92 | Me | Cl | F | H | F | F | H | 1,3-Thiazol-2-yl | |
| 93 | Me | Cl | F | H | F | F | H | Pyrimidin-2-yl | |
| 94 | Me | Cl | F | H | H | H | Cl | 5-Chlor-2-thienyl | 4,16 |
| 95 | Me | Cl | F | H | H | H | Cl | 5,6-Dimethylpyridin-2-yl | |
| 96 | Me | Cl | F | H | H | H | Cl | Pyridin-2-yl | |
| 97 | Me | Cl | F | H | H | H | Cl | 5-Methyl-2-furyl | |
| 98 | Me | Cl | F | H | H | H | Cl | 1,3-Thiazol-2-yl | |
| 99 | Me | Cl | F | H | H | H | Cl | Pyrimidin-2-yl | |
| 100 | Me | Cl | F | H | H | H | F | 2-Thienyl | 3,12 |
| 101 | Me | Cl | F | H | H | H | F | 5,6-Dimethylpyridin- 2-yl | 2,88 |
| 102 | Me | Cl | F | H | H | H | F | Pyridin-4-yl | 2,08 |
| 103 | Me | Cl | F | H | H | H | F | 5-Chlor-2-thienyl | 3,84 |
| 104 | Me | Cl | F | H | H | H | F | 5-Methyl-2-furyl | 3,26 |
| 105 | Me | Cl | F | H | H | H | F | 1,3-Thiazol-2-yl | 2,51 |
| 106 | Me | Cl | F | H | H | H | F | Pyridin-2-yl | |
| 107 | Me | Cl | F | H | H | H | F | Pyridin-3-yl | |
| 108 | Me | Cl | F | H | H | H | F | Pyrimidin-2-yl | |
| 109 | Me | Cl | F | F | H | H | F | 2-Thienyl | |
| 110 | Me | Cl | F | F | H | H | F | 5-chlor-2-thienyl | |
| 111 | Me | Cl | F | F | H | H | F | 5,6-Dimethylpyridin-2-yl | |
| 112 | Me | Cl | F | F | H | H | F | Pyridin-4-yl | |
| 113 | Me | Cl | F | F | H | H | F | Pyridin-2-yl | |
| 114 | Me | Cl | F | F | H | H | F | Pyridin-3-yl | |
| 115 | Me | Cl | F | F | H | H | F | 5-Methyl-2-furyl | |
| 116 | Me | Cl | F | F | H | H | F | 1,3-thiazol-2-yl | |
| 117 | Me | Cl | F | F | H | H | F | Pyrimidin-2-yl | |
| 118 | Me | Cl | F | H | H | H | H | 5-Chlor-2-thienyl | 3,7 |
| 119 | Me | Cl | F | H | H | F | H | 5-Chlor-2-thienyl | 3,42 |
| 120 | Me | Cl | F | H | H | Cl | H | 5-Chlor-2-thienyl | 4,16 |
| 121 | Me | Cl | F | H | H | H | H | 2-Thienyl | 3,05 |
| 122 | Me | Cl | F | H | H | H | H | 5,6-Dimethylpyridin-2-yl | |
| 123 | Me | Cl | F | H | H | H | H | Pyridin-4-yl | |
| 124 | Me | Cl | F | H | H | H | H | Pyridin-2-yl | |
| 125 | Me | Cl | F | H | H | H | H | Pyridin-3-yl | |
| 126 | Me | Cl | F | H | H | H | H | 5-Methyl-2-furyl | |
| 127 | Me | Cl | F | H | H | H | H | 1,3-Thiazol-2-yl | |
| 128 | Me | Cl | F | H | H | H | H | Pyrimidin-2-yl | |
| 129 | Me | Cl | F | H | H | F | H | 2-Thienyl | |
| 130 | Me | Cl | F | H | H | F | H | 5,6-Dimethylpyridin-2-yl | |
| 131 | Me | Cl | F | H | H | F | H | Pyridin-4-yl | |
| 132 | Me | Cl | F | H | H | F | H | Pyridin-2-yl | |
| 133 | Me | Cl | F | H | H | F | H | Pyridin-3-yl | |
| 134 | Me | Cl | F | H | H | F | H | 5-Methyl-2-furyl | |
| 135 | Me | Cl | F | H | H | F | H | 1,3-Thiazol-2-yl | |
| 136 | Me | Cl | F | H | H | F | H | Pyrimidin-2-yl | |
| 137 | Me | Cl | F | F | H | F | H | 2-Thienyl | |
| 138 | Me | Cl | F | F | H | F | H | 5-Chlor-2-thienyl | |
| 139 | Me | Cl | F | F | H | F | H | 5,6-Dimethylpyridin-2-yl | |
| 140 | Me | Cl | F | F | H | F | H | Pyridin-4-yl | |
| 141 | Me | Cl | F | F | H | F | H | Pyridin-2-yl | |
| 142 | Me | Cl | F | F | H | F | H | Pyridin-3-yl | |
| 143 | Me | Cl | F | F | H | F | H | 5-Methyl-2-furyl | |
| 144 | Me | Cl | F | F | H | F | H | 1,3-Thiazol-2-yl | |
| 145 | Me | Cl | F | F | H | F | H | Pyrimidin-2-yl | |
| 146 | Me | Cl | F | H | H | Cl | H | 2-Thienyl | |
| 147 | Me | Cl | F | H | H | Cl | H | 5,6-Dimethylpyridin-2-yl | |
| 148 | Me | Cl | F | H | H | Cl | H | Pyridin-4-yl | |
| 149 | Me | Cl | F | H | H | Cl | H | Pyridin-2-yl | |
| 150 | Me | Cl | F | H | H | Cl | H | Pyridin-3-yl | |
| 151 | Me | Cl | F | H | H | Cl | H | 5-Methyl-2-furyl | |
| 152 | Me | Cl | F | H | H | Cl | H | 1,3-Thiazol-2-yl | |
| 153 | Me | Cl | F | H | H | Cl | H | Pyrimidin-2-yl | |
| 154 | Me | Cl | H | H | F | H | H | 2-Thienyl | |
| 155 | Me | Cl | H | H | F | H | H | 5-Chlor-2-thienyl | |
| 156 | Me | Cl | H | H | F | H | H | 5,6-Dimethylpyridin-2-yl | |
| 157 | Me | Cl | H | H | F | H | H | Pyridin-4-yl | |
| 158 | Me | Cl | H | H | F | H | H | Pyridin-2-yl | |
| 159 | Me | Cl | H | H | F | H | H | Pyridin-3-yl | |
| 160 | Me | Cl | H | H | F | H | H | 5-Methyl-2-furyl | |
| 161 | Me | Cl | H | H | F | H | H | 1,3-Thiazol-2-yl | |
| 162 | Me | Cl | H | H | F | H | H | Pyrimidin-2-yl | |
| 163 | Me | Cl | H | H | H | H | CN | 2-Thienyl | |
| 164 | Me | Cl | H | H | H | H | CN | 5-Chlor-2-thienyl | |
| 165 | Me | Cl | H | H | H | H | CN | 5,6-Dimethylpyridin-2-yl | |
| 166 | Me | Cl | H | H | H | H | CN | Pyridin-4-yl | |
| 167 | Me | Cl | H | H | H | H | CN | Pyridin-2-yl | |
| 168 | Me | Cl | H | H | H | H | CN | Pyridin-3-yl | |
| 169 | Me | Cl | H | H | H | H | CN | 5-Methyl-2-furyl | |
| 170 | Me | Cl | H | H | H | H | CN | 1,3-Thiazol-2-yl | |
| 171 | Me | Cl | H | H | H | H | CN | Pyrimidin-2-yl | |
| 172 | Me | Cl | H | Cl | H | H | H | 2-Thionyl | 3,34 |
| 173 | Me | Cl | H | Cl | H | H | H | 5-Chlor-2-thienyl | 4,07 |
| 174 | Me | Cl | H | Cl | H | H | H | 5,6-Dimethylpyridin-2-yl | |
| 175 | Me | Cl | H | Cl | H | H | H | Pyridin-4-yl | |
| 176 | Me | Cl | H | Cl | H | H | H | Pyridin-2-yl | |
| 177 | Me | Cl | H | Cl | H | H | H | Pyridin-3-yl | |
| 178 | Me | Cl | H | Cl | H | H | H | 5-Methyl-2-furyl | |
| 179 | Me | Cl | H | Cl | H | H | H | 1,3-Thiazol-2-yl | |
| 180 | Me | Cl | H | Cl | H | H | H | Pyrimidin-2-yl | |
| 181 | Me | Cl | MeO | H | H | H | H | 2-Thienyl | |
| 182 | Me | Cl | MeO | H | H | H | H | 5-Chlor-2-thienyl | 3,65 |
| 183 | Me | Cl | MeO | H | H | H | H | 5,6-Dimethylpyridin-2-yl | |
| 184 | Me | Cl | MeO | H | H | H | H | Pyridin-4-yl | |
| 185 | Me | Cl | MeO | H | H | H | H | Pyridin-2-yl | |
| 186 | Me | Cl | MeO | H | H | H | H | Pyridin-3-yl | |
| 187 | Me | Cl | MeO | H | H | H | H | 5-Methyl-2-furyl | |
| 188 | Me | Cl | MeO | H | H | H | H | 1,3-Thiazol-2-yl | |
| 189 | Me | Cl | MeO | H | H | H | H | Fyrimidin-2-yl | |
| 190 | Me | Cl | Me | H | H | H | H | 2-Thienyl | 3,18 |
| 191 | Me | Cl | Me | H | H | H | H | 5-Chlor-2-thienyl | |
| 192 | Me | Cl | Me | H | H | H | H | 5,6-Dimethylpyridin-2-yl | |
| 193 | Me | Cl | Me | H | H | H | H | Pyridin-4-yl | |
| 194 | Me | Cl | Me | H | H | H | H | Pyridin-2-yl | |
| 195 | Me | Cl | Me | H | H | H | H | Pyridin-3-yl | |
| 196 | Me | Cl | Me | H | H | H | H | 5-Methyl-2-furyl | |
| 197 | Me | Cl | Me | H | H | H | H | 1,3-Thiazol-2-yl | |
| 198 | Me | Cl | Me | H | H | H | H | Pyrimidin-2-yl | |
| 199 | Me | Cl | Me | H | Me | H | Me | 2-Thienyl | |
| 200 | Me | Cl | Me | H | Me | H | Me | 5-Chlor-2-thienyl | |
| 201 | Me | Cl | Me | H | Me | H | Me | 5,6-Dimethylpyridin-2-yl | |
| 202 | Me | Cl | Me | H | Me | H | Me | Pyridin-4-yl | |
| 203 | Me | Cl | Me | H | Me | H | Me | Pyridin-2-yl | |
| 204 | Me | Cl | Me | H | Me | H | Me | Pyridin-3-yl | |
| 205 | Me | Cl | Me | H | Me | H | Me | 5-Methyl-2-furyl | |
| 206 | Me | Cl | Me | H | Me | H | Me | 1,3-Thiazol-2-yl | |
| 207 | Me | Cl | Me | H | Me | H | Me | Pyrimidin-2-yl | |

| | | | | | | | | | |
|---|---|---|---|---|---|---|---|---|---|
| ^{a)} Die Bestimmung der IogP Werte erfolgte gemäß EEC Directive 79/831 Annex V.A8 durch HPLC (High Performance Liquid Chromatography) an reversed-phase Säulen (C 18), mit nachfolgenden Methoden: * Temperatur: 40°C , Mobile Phase : 0.1% wässrige Ameisensäure und Acetonitril ; Linearer Gradient von 10% Acetonitril bis 95% Acetonitril. **Temperatur: 43°C ; Mobile Phase : 0.1% wässrige Phosphorsäure und Acetonitril ; Linearer Gradient von 10% Acetonitril bis 95% Acetonitril. | | | | | | | | | |

Die Kalibrierung wurde jeweils mit unverzweigten Alkan-2-onn (3 bis 16 Kohlenstoff Atome) mit bekannten IogP Werten durchgeführt (Bestimmung von den logP Werten über die Retentionszeiten durch lineare Interpolation zwischen zwei bestimmten Alkazionen). Die Lambda-max Werte wurden jeweils in den Maxima der Chromatographie-Signale durch UV Spektren zwischen 190nm und 400nm bestimmt.

### Verwendungsbeispiele

### Beispiel A

### Venturia - Test (Apfel) / protektiv

| | |
|---|---|
| Lösungsmittel: | 24,5 Gewichtsteile Aceton |
| | 24,5 Gewichtsteile Dimethylacetamid |
| Emulgator: | 1 Gewichtsteil Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wäßrigen Konidiensuspension des Apfelschorferregers ***Venturia inaequalis*** inokuliert und verbleiben dann 1 Tag bei ca. 20°C und 100% relativer Luftfeuchtigkeit in einer Inkubations-kabine.

Die Pflanzen werden dann im Gewächshaus bei ca, 21°C und einer relativen Luftfeuchtigkeit von ca. 90% aufgestellt.

10 Tage nach der Inokulation erfolgt die Auswertung. Dabei bedeutet 0% ein Wirkungsgrad, der demjenigen der Kontrolle entspricht, während ein Wirkungsgrad von 100% bedeutet, daß kein Befall beobachtet wird.

In diesem Test zeigen die erfindungsgemäßen Verbindungen der nachfolgenden Formeln bei einer Konzentration an Wirkstoff von 100 ppm einen Wirkungsgrad von 70% oder mehr:
Beispiele Nr, 37, Nr. 94, Nr. 100, Nr. 101, Nr. 103 gemäß Tabelle 1.

### Beispiel B

### Alternaria-Test (Tomate) / protektiv

| | |
|---|---|
| Lösungsmittel: | 24,5 Gewichtsteile Aceton |
| | 24,5 Gewichtsteile Dimethylacetamid |
| Emulgator : | 1 Gewichtsteil Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wäßrigen Sporensuspension von ***Alternaria solani*** inokuliert. Die Pflanzen werden dann in einer Inkubationskabine bei ca. 20°C und 100% relativer Luftfeuchtigkeit aufgestellt.

3 Tage nach der Inokulation erfolgt die Auswertung, Dabei bedeutet 0% ein Wirkungsgrad, der demjenigen der Kontrolle entspricht, während ein Wirkungsgrad von 100% bedeutet, daß kein Befall beobachtet wird.

In diesem Test zeigen die erfindungsgemäßen Verbindungen der nachfolgenden Formeln bei einer Konzentration an Wirkstoff von 100ppm einen Wirkungsgrad von 70% oder mehr.

Beispiele Nr. 37, Nr, 94, Nr. 100, Nr. 101, Nr. 103 gemäß Tabelle 1,

### Beispiele

### Botrytis - Test (Bohne) / protektiv

| | |
|---|---|
| Lösungsmittel: | 24,5 Gewichtsteile Aceton |
| | 24,5 Gewichtsteile Dimethylacetamid |
| Emulgator : | 1 Gewichtsteil Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoff-zubereitung in der angegebenen Aufwandmenge besprüht. Nach Antrocknen des Spritzbelages werden auf jedes Blatt 2 kleine mit ***Botrytis cinerea*** bewachsene Agarstückchen aufgelegt. Die inokulierten Pflanzen werden in einer abgedunkelten Kammer bei ca. 20°C und 100% relativer Luftfeuchtigkeit aufgestellt.

2 Tage nach der Inokulation wird die Größe der Befallsflecken auf den Blättern ausgewertet. Dabei bedeutet 0% ein Wirkungsgrad, der demjenigen der Kontrolle entspricht, während ein Wirkungsgrad von 100% bedeutet, daß kein Befall beobachtet wird.

In diesem Test zeigen die erfindungsgemäßen Verbindungen der nachfolgenden Formeln bei einer Konzentration an Wirkstoff von 250 ppm einen Wirkungsgrad von 70% oder mehr.
Beispiele Nr. 37, Nr. 94, Nr; 100, Nr. 103 gemäß Tabelle 1.

### Beispiel D

### Sphaerotheca-Test (Gurke) / protektiv

| | | |
|---|---|---|
| Lösungsmittel- | 49 Gewichtsteile | N, N - Dimethylformamid |
| Emulgator: | 1 Gewichtsteil | Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Gurkenpflanzen mit der Wirkstoffzubereitung in der angegebenen Aufwandmenge, 1 Tag nach der Behandlung werden die Pflanzen mit einer Sporensuspension von ***Sphaerotheca fuliginea*** inokuliert, Anschließend werden die Pflanzen in einem Gewächshaus bei 70 % relativer Luftfeuchtigkeit und einer Temperatur von 23°C aufgestellt.

7 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, daß kein Befall beobachtet wird,

In diesem Test zeigen die erfindungsgemäßen Verbindungen der nachfolgenden Formeln bei einer Konzentration an Wirkstoff von 500 ppm einen Wirkungsgrad von 70% oder mehr.
Beispiele Nr. 37, Nr. 94, Nr, 100, Nr. 101, Nr. 190 gemäß Tabelle 1.

### Beispiel E

### Erysiphe-Test (Gerste) / protektiv

| | |
|---|---|
| Lösungsmittel: | 49 Gewichtsteile N,N-Dimethylacetamid |
| Emulgator: | 1 Gewichtsteil Alkylmylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung in der angegebenen Aufwandmenge.

Nach Antrocknen des Spritzbelages werden die Pflanzen mit Sporen von ***Erysiphe graminis f.sp, hordei*** bestäubt.

Die Pflanzen werden in einem Gewächshaus bei einer Temperatur von ca. 20°C und einer relativen Luftfeuchtigkeit von ca. 80 % aufgestellt, um die Entwicklung von Mehltaupusteln zu begünstigen.

7 Tage nach der Inokulation erfolgt die Auswertung, Dabei bedeutet 0 % ein Wirkungsgrad, der demjenigen der Kontrolle entspricht, während ein Wirkungsgrad von 100 % bedeutet, daß kein Befall beobachtet wird.

In diesem Test zeigen die erfindungsgemäßen nachfolgenden Verbindungen bei einer Konzentration an Wirkstoff von 1000 ppm einen Wirkungsgrad von 70% oder mehr.
Beispiel Nr. 100 gemäß Tabelle 1.

### Beispiel F

### Leptosphaeria nodorum-Test (Weizen) / protektiv

| | |
|---|---|
| Lösungsmittel: | 49 Gewichtsteile N,N-Dimethylacetamid |
| Emulgator; | 1 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration,

Zur Prüfung auf protektive Wirksamkeit werden junge Pflanzen mit der Wirkstoffzubereitung in der angegebenen Aufwandmenge besprüht Nach Antrocknen des Spritzbelages werden die Pflanzen mit einer Sporensuspension von ***Leptosphaeria nodorum*** besprüht, Die Pflanzen verbleiben 48 Stunden bei 20°C und 100 % relativer Luftfeuchtigkeit in einer Inkubationskabine.

Die Pflanzen werden in einem Gewächshaus bei einer Temperatur von ca. 20°C und einer relativen Luftfeuchtigkeit von 80 % aufgestellt.

10 Tage nach der Inokulation erfolgt die Auswertung. Dabei bedeutet 0% ein Wirkungsgrad, der demjenigen der Kontrolle entspricht, während ein Wirkungsgrad von 100% bedeutet, daß kein Befall beobachtet wird.

In diesem Test zeigen die erfindungsgemäßen nachfolgenden Verbindungen bei einer Konzentration an Wirkstoff von 1000 ppm einen Wirkungsgrad von 70% oder mehr.
Beispiel Nr. 100 gemäß Tabelle 1.

### Beispiel G

### Pyrenophora teres-Test (Gerste) / protektiv

| | |
|---|---|
| Lösungsmittel: | 49 Gewichtsteile N,N-Dimethylacetamid |
| Emulgator; | 1 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegeben Aufwandmenge besprüht, Nach Antrocknen des Spritzbelages werden die Pflanzen mit einer Konidiensuspension von ***Pyrenophora teres*** besprüht. Die Pflanzen verbleiben 48 Stunden bei 20°C und 100 % relativer Luftfeuchtigkeit in einer Inkubationskabine.

Die Pflanzen werden dann in einem Gewächshaus bei einer Temperatur von ca. 20°C und relativen Luftfeuchtigkeit von ca. 80 % aufgestellt.

8 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, daß kein Befall beobachtet wird,

In diesem Test zeigen die erfindungsgemäßen nachfolgenden Verbindungen bei einer Konzentration an Wirkstoff von 1000 ppm einen Wirkungsgrad von 70% oder mehr.
Beispiel Nr, 100 gemäß Tabelle 1,

### Beispiel H

### Fusarium nivale (var. majus)-Test (Weizen) / protektiv

| | |
|---|---|
| Lösungsmittel: | 49 Gewichtsteile N,N-Dimethylacetamid |
| Emulgator: | 1,0 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration,

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer Konidiensuspension von ***Fusarium nivale (var. majus)*** besprüht.

Die Pflanzen werden in einem Gewächshaus unter lichtdurchlässigen Inkubationshauben bei einer Temperatur von ca.15°C und einer relativen Luftfeuchtigkeit von ca. 100 % aufgestellt.

6 Tage nach der Inokulation erfolgt die Auswertung. Dabei bedeutet 0% ein Wirkungsgrad, der demjenigen der Kontrolle entspricht, während ein Wirkungsgrad von 100% bedeutet, daß kein Befall beobachtet wird.

In diesem Test zeigen die erfindungagemäße Verbindung der nachfolgenden Formel bei einer Konzentration an Wirkstoff von 1000 ppm einen Wirkungsgrad von 70% oder mehr.
Beispiel Nr, 100 gemäß Tabelle 1,

## Patentansprüche

**1.** Verbindungen der Formel (I), 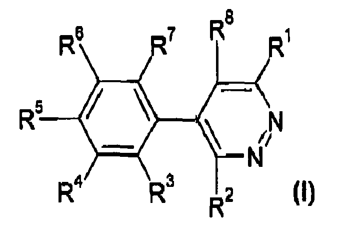 in welcher die Symbole folgende Bedeutung haben:
R¹ steht für C₁-C₈-Alkyl, C₂-C₈-Alkenyl, C₁-C₈-Alkinyl, C₃-C₈-Cycloalkyl, C₃-C₈-Cycloalkenyl oder für einen drei- bis zehngliedrigen gesättigten, mono- oder bicyclischen C-gebundenen Heterocyclus, enthaltend ein bis vier Heteroatome aus der Gruppe O, N oder S, mit der Maßgabe dass zwei Sauerstoffatome nicht benachbart sein dürfen, und wobei R¹ durch eine bis drei gleiche oder verschiedene Gruppen R^{a} substituiert sein kann und
R^{a} steht für Halogen, Alkylamino, Dialkylamino, Alkylhydrazino, Cyano, Oxo, Nitro, C₁-C₆-Alkylthio, C₁-C₆-Alkyl, C₁-C₆-Halogenalkyl, tri(C₁-C₄-Alkyl)silyl, und/oder C₁-C₆-Alkoxy und/oder für unsubstituiertes, durch Halogen, C₁-C₄-Alkyl oder C₁-C₄-Halogenalkyl substituiertes C₃-C₆-Cycloalkyl,
R² steht für Halogen, Cyano, jeweils geradkettiges oder verzweigtes C₁-C₆-Alkoxy, C₁-C₆-Alkylthio, C₁-C₆-Alkylsulfinyl, C₁-C₆-Alkylsulfonyl, C₁-C₆-Halogenalkoxy oder C₁-C₆-Halogonalkylthio,
R³ bis R⁷ stehen unabhängig voneinander gleichartig oder verschieden für
Wasserstoff, Halogen, Cyano, Nitro, Amino, Hydroxy, Mercapto, Formyl, Carboxy, Carbamoyl, Thiocarbamoyl;
jeweils geradkettiges oder verzweigtes Alkyl, Alkoxy, Alkylthio, Alkylsulfinyl oder Alkylsulfonyl mit jeweils 1 bis 6 Kohlenstoffatomen;
jeweils geradkettiges oder verzweigtes Alkenyl oder Alkenyloxy mit jeweils 2 bis 6 Kohlenstoffatomen;
jeweils geradkettiges oder verzweigtes Alkinyl oder Alkinyloxy mit jeweils 2 bis 6 Kohlenstoffatomen;
jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen;
jeweils geradkettiges oder verzweigtes Halogenalkenyl oder Halogenalkenyloxy mit jeweils 2 bis 6 Kohlenstoffatomen und 1 bis 11 gleichen oder verschiedenen Halogenatomen oder geradkettiges oder verzweigtes Halogenalkinyloxy mit 2 bis 6 Kohlenstoffatomen;
jeweils geradkettiges oder verzweigtes Alkylamino, Dialkylamino, Alkylcarbonyl, Alkylcarbonyloxy, Alkoxycarbonyl, Alkylsulfonyloxy, Hydroximinoalkyl oder Alkoximinoalkyl mit jeweils 1 bis 6 Kohlenstoffatomen in den einzelnen Alkylteilen;
jeweils geradkettiges oder verzweigtes Alkoxyalkyl, Halogenalkoxyalkyl, Alkylaminoalkyl, Dialkylaminoalkyl mit jeweils 1 bis 6 Kohlenstoffatomen in den einzelnen Alkylteilen;
jeweils geradkettiges oder verzweigtes Alkoxyalkoxy, Alkoxyalkoxyalkoxy, Halogenalkoxyalkoxy, Alkylaminoalkoxy, Dialkylaminoalkoxy mit jeweils 1 bis 6 Kohlenstoffatomen in den einzelnen Alkylteilen;
Cycloalkyl mit 3 bis 8 Kohlenstoffatomen;
Oder zwei benachbarte Reste R³ bis R⁷ bilden zusammen einen fünf- bis siebengliedrigen gesättigten, teilweise gesättigten oder ungesättigten substituierten oder unsubstituierten Ring ggf. enthaltend bis zu zwei Heteroatome ausgewählt aus Stickstoff, Sauerstoff oder Schwefel, mit der Maßgabe dass zwei Sauerstoffatome nicht benachbart sein dürfen;
R⁸ steht für ganz oder teilweise ungesättigtes Heterocyclyl mit fünf bis sieben Ringgliedern und ein bis vier Heteroatomen aus der Gruppe Stickstoff, Sauerstoff und Schwefel, mit der Maßgabe dass zwei Sauerstoffatome nicht benachbart sein dürfen, gegebenenfalls annelliert mit einem weiteren fünf- bis sechsgliedrigen gesättigten oder ganz oder teilweise ungesättigten carbocyclischen oder heterocyclischen Ring, wobei der heterocyclische Ring ggf. ein bis zwei zusätzliche Heteroatome aus der Gruppe Stickstoff, Sauerstoff und Schwefel enthalten kann, mit der Maßgabe dass zwei Sauerstoffatome nicht benachbart sein dürfen, wobei R⁸ einfach oder mehrfach gleichartig oder verschieden durch R⁹ substituiert ist, und
R⁹ steht für Wasserstoff, Halogen, Cyano, Amino, Hydroxy, Mercapto, Formyl, Carboxy, Carbamoyl, Thiocarbamoyl;
jeweils geradkettiges oder verzweigtes Alkyl, Alkoxy oder Alkylthio mit jeweils 1 bis 6 Kohlenstoffatomen;
jeweils geradkettiges oder verzweigtes Halogenalkyl, Halogenalkoxy oder Halogenalkylthio mit jeweils 1 bis 6 Kohlenstoffatomen und 1 bis 13 gleichen oder verschiedenen Halogenatomen;
jeweils geradkettiges oder verzweigtes Alkylamino, Dialkylamino, Alkylcarbonyl, Alkylcarbonyloxy, Alkoxycarbonyl, Hydroximinoalkyl oder Alkoximinoalkyl mit jeweils 1 bis 6 Kohlenstoffatomen in den einzelnen Alkylteilen;
jeweils geradkettiges oder verzweigtes Alkoxyalkyl, Halogenalkoxyalkyl, Alkylaminoalkyl, Dialkylaminoalkyl mit jeweils 1 bis 6 Kohlenstoffatomen in den einzelnen Alkylteilen;
jeweils geradkettiges oder verzweigtes Alkoxyalkoxy, Alkoxyalkoxyalkoxy, Halogenalkoxyalkoxy, Alkylaminoalkoxy, Dialkylaminoalkoxy mit jeweils 1 bis 6 Kohlenstoffatomen in den einzelnen Alkylteilen;
unsubstituiertes oder einfach bis fünffach substituiertes Cycloalkyl mit 3 bis 8 Kohlenstoffatomen, wobei die Substituenten ausgewählt aus Fluor, Chlor, Methyl oder Trifluormethyl unabhängig voneinander gleich oder verschieden sein können;
oder zwei Reste R⁹ bilden mit dem Kohlenstoffatom, an das sie gebundenen sind, eine Carbonyl- oder Thiocarbonylgruppe,
sowie agrochemisch wirksame Salze davon.

**2.** Verbindungen der Formel (I) gemäß Anspruch 1, **dadurch gekennzeichnet, dass** die Symbole folgende Bedeutung haben:
R¹ steht für Wasserstoff, Methyl, Ethyl, n-Propyl, Isopropyl, Cyclopropyl, n-Butyl, Isobutyl, sek-Butyl, tert-Butyl, Methoxymethyl, Chlordifluormethyl, Dichlorfluormethyl, Chlorfluormethyl , Chlormethyl, Dichlormethyl, Trichlormethyl, Fluormethyl, Difluormethyl, Trifluormethyl, 2.2.2-Trifluorethyl;
R² steht für Fluor, Chlor, Brom, Cyano, Methoxy, Ethoxy, n-Propoxy, Isopropoxy, Methylthio, Ethylthio, n-Propylthio, Isopropylthio, Methylsulfinyl, EthylsulFinyl, Methylsulfonyl, Ethylsulfonyl, Fluormethoxy, Difluormethoxy, Trifluormethoxy, Trifluormethylthio, 2.2.2-Trifluorethoxy;
R³ bis R⁷ stehen unabhängig voneinander gleichartig oder verschieden für
Wasserstoff, Fluor, Chlor, Brom, Jod, Cyano, Nitro, Hydroxy, Mercapto, Amino, Formyl, Methyl, Ethyl, n-Propyl, Isopropyl, n-Butyl, Isobutyl, sek-Butyl, tert-Butyl, Chlordifluormethyl, Dichlorfluormethyl, Chlorfluormethyl, Chlormethyl, Dichlormethyl, Trichlormethyl, Fluormethyl, Difluormethyl, Trifluormethyl, 2.2-Dichlor-2-fluoroethyl, 2-Chlor-2.2-difluorethyl, 2-Chlor-2-fluorethyl, 2.2.2-Trichlorethyl, 2-Fluorethyl, 2.2-Difluorethyl, 2.2.2-Trifluorethyl, Vinyl, Ethinyl, Allyl, Propargyl, Methoxy, Ethoxy, n- oder i-Propoxy, Methylthio, Ethylthio, n-Propylthio, Isopropylthio, Methylsulfinyl, Ethylsulfinyl, Methylsulfonyl, Ethylsulfonyl, Allyloxy, Propargyloxy, Difluormethoxy, Trifluormethoxy, Difluorchlormethoxy, 2.2.2-Trifluorethoxy, Difluormethylthio, Difluorchlormethylthio, Trifluormethylthio, Trifluormethylsulfinyl, Trifluormethylsulfonyl, Trichlorethinyloxy, Trifluorethinyloxy, Chlorallyloxy, Methylamino, Ethylamino, n-Propylamino, Isopropylamino, Cyclopropylamino, Dimethylamino, Diethylamino, Acetyl, Propionyl, Acetyloxy, Methoxycarbonyl, Ethoxycarbonyl, Methoxymethyl, Ethoxymethyl, 2.Methoxyethoxy, 2-Ethoxyethoxy, (2-Methoxyethoxy)ethoxy, 2-(Dimethylamino)ethoxy, 2-Morpholin-4-ylethoxy, 3-(Dimethylamino)propoxy, 3-Morpholin-4-ylpropoxy, Hydroximinomethyl, Hydroximinoethyl, Methoximinomethyl, Ethoximinomethyl, N-Methoxyethanimidoyl, N-Ethoxyethanitnidoyl, Cyclopropyl, Cyclobutyl, Cyclopentyl, Cyclohexyl;
Oder zwei benachbarte Reste R³ bis R⁷ bilden zusammen eine 1,3-Propandiyl-, 1,4-Butandiyl-, Methylenbis(oxy)- oder Ethan-1,2-diylbis(oxy)-Gruppe, wobei diese Reste einfach oder mehrfach, gleichartig oder verschieden substituiert sein können durch Fluor, Chlor, Methyl, Ethyl, n-Propyl, Isopropyl und/oder Trifluormethyl;
Wobei bis zu vier Reste R³ bis R⁷ ungleich Wasserstoff sein können;
R⁸ steht bevorzugt für einen der nachstehend genannten heterocylischen Reste, welcher jeweils unsubstituiert oder einfach oder mehrfach durch gleiche oder verschiedenene Reste R⁹ substituiert sein kann:
2-Furyl, 3-Furyl, 2-Thienyl, 3-Thienyl, 1-Pyrrolyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 1-pyrazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 1-Imidazolyl, 2-Imidazolyl, 4-Imidazolyl, 5-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,3,4-Oxadiazot-2-yl, 1,3,4-Thiadiazol-2-yl, 1,2,4-Triazol-1-yl, 1,2,4-Triazol-3-yl, 1,2,4-Triazol-4-yl, 1,2,4-Triazol-5-yl, 1,2,3-Triazol-1-yl, 1,2,3-Triazol-2-yl, 1,2,3-Triazol-4-yl, Trtrazol-1-yl, Tetrazol-2-yl, Tetrazol-5-yl, Indol-1-yl, Indol-2-yl, Indol-3-yl, Isoindol-1-yl, Isoindol-2-yl, Benzofur-2-yl, Benzothiophen-2-yl, Benzofur-3-yl, Benzothiophen-3-yl, Benzoxazol-2-yl, Benzothiazol-2-yl, Benzimidazol-2-yl, Indazol-1-yl, Indazol-2-yl, Indazol-3-yl, 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl, 1,2,4-Triazin-3-yl, 1,2,4-Triazin-5-yl, 1,2,4-Triazin-6-yl;
R⁹ ist gleich oder verschieden Wasserstoff, Fluor, Chlor, Brom, Iod, Cyano, Hydroxy, Mercapto, Amino, Methyl, Ethyl, n-Propyl, Isopropyl, n-Butyl, Isobutyl, sek-Butyl, tert-Butyl, Cyclopropyl, 1-Chlorcyclopropyl, Methoxy, Ethoxy, Isopropoxy, Methylthio, Ethylthio, Trifluormethylthio, Chlordifluormethyl, Dichlorfluormethyl, Chlorfluormethyl, Chlormethyl, Dichlormethyl, Trichlormethyl, Fluormethyl, Difluormethyl, Trifluormethyl, 2.2.2-Trifluorethyl, Trifluormethoxy, Trifluormethylthio, 2.2,2-Trifluorothoxy, 2,2-Dichlor-2-fluorethyl, 2.2-Difluor-2-chlorethyl, 2-Chlor-2-fluorethyl, 2.2.2-Trichlorothyl, 2.2.2-Trifluorethyl, 2-Fluorethyl, 2.2-Difluorethyl, 2-Methoxyethoxy, Acetyl, Propionyl, Methoxycarbonyl, Ethoxycarbonyl, N-Methylamino, N,N--Dimethylammo, N-Ethylamino, N,N-Diethylamino;
oder zwei Reste R⁹ bilden mit dem Kohlenstoffatom, an das sie gebundenen sind, eine Carbonyl- oder Thiocarbonylgruppe,
sowie agrochemisch wirksame Salze davon.

**3.** Verbindungen der Formel (I) gemäß einem oder mehreren der Ansprüche 1 bis 2, **dadurch gekennzeichnet,, dass** die Symbole folgende Bedeutung haben:
R₁ steht für Methyl, Ethyl, Cyclopropyl, Fluormethyl, Difluormethyl, Trifluormethyl;
R² steht für Fluor, Chlor, Brom, Cyano;
R³ steht für Fluor, Chlor, Brom, Cyano, Methyl, Difluormethyl, Trifluormethyl, Trifluormethoxy, Methoxy;
R⁴ bis R⁷ stehen unabhängig voneinander gleichartig oder verschieden für:
Wasserstoff, Fluor, Chlor, Brom, Iod, Cyano, Methyl, Ethyl, n-Propyl, Isopropyl, Isobutyl, tert-Butyl, Cyclopropyl, Methoxy, Ethoxy, Methylthio, Difluormethyl, Trifluormethyl, 2.2.2-Trifluorethyl, Trifluormethoxy, Trifluormethylthio, 2-Methoxyethoxy, (2-Methoxyethoxy)ethoxy, 2-(Dimethylamino)ethoxy, 2-Morpholin-4-ylethoxy, 3-(Dimethylamino)propoxy, 3-Morpholin-4-ylpropoxy;
oder zwei benachbarte Reste R³ bis R⁷ bilden zusammen eine Methylenbis(oxy)- oder Ethan-1,2-diylbis(oxy)-Gruppe, wobei diese Reste einfach oder mehrfach, gleichartig oder verschieden durch Fluor oder Methyl substituiert sein können;
wobei bis zu drei Reste R⁴ bis R⁷ ungleich Wasserstoff sein können; .
R⁸ steht für einen der nachstehend genannten heterocylischen Reste, welcher jeweils unsubstituiert oder einfach oder mehrfach durch gleiche oder verschiedenene Reste R⁹ substituiert sein kann:
2-Furyl, 3-Furyl, 2-Thienyl, 3-Thienyl, 1-Pyrrolyl, 2-Pyrrolyl, 3-Pyrrolyl, 3-Isoxazolyl, 4-Isoxazolyt, 5-Isoxazolyl, 3-Isothiazolyl, 4-Isothiazolyl, 5-Isothiazolyt, 1-Pyrazolyl, 3-Pyrazolyl, 4-Pyrazolyl, 5-Pyrazolyl, 2-Oxamlyl, 4-Oxazolyl, 5-Oxazolyl, 2-Thiazolyl, 4-Thiazolyl, 5- Thiazolyl, 1-Imidazolyl, 2-Imidazolyl, 4-Imidazolyl, 5-Ymidazolyl, 1,2,4-Oxadiazol-3-y1, 1,2,4-Oxadiazol-5-yl, 1,3,4-Oxadiazol-2-yl, 1,2,4-Thiadiazol-3-yl, 1,2,4-Thiadiezol-5-yl, 1,3,4-Thiadiazol-2-yl, 1,2,4 Triazol-1-yl, 1,2,4-Triazol-3-yl, 1,2,4-Triazol-4-yl, 1,2,4-Triazol-5-yl, Tetmzol-5-yl, 2-Pyridinyl, 3-Pyridinyl, 4-Pyridinyl, 3-Pyridazinyl, 4. Pyridazinyl, 2-Pyrimidinyl, 4-Pyrimidinyl, 5-Pyrimidinyl, 2-Pyrazinyl, 1,3,5-Triazin-2-yl, 1,2,4-Triazin-3-yl, 1,2,4-Triazin-5-yl, 1,2,4-Triazin-6-yl;
R⁹ ist gleich oder verschieden Wasserstoff, Fluor, Chlor, Brom, Iod, Cyano, Hydroxy, Mercapto, Methyl, Ethyl, n-Propyl, Isopropyl, n-Butyl, Isobutyl, sek-Butyl, tert.-Butyl, Cyclopropyl, Methoxy, Ethoxy, Methylthio, Ethylthio, Trifluormethylthio, Chlordifluormethyl, Dichlorfluormethyl, Chlorfluormethyl, Chlormethyl, Dichlormethyl, Trichlormethyl, Difluormethyl, Trifluormethyl , 2.2.2-Trifluorethyl, Trifluormethoxy, 2.2.2-Trifiuorethoxy, 2-Mothoxyethoxy, Acetyl, Propionyl, Methoxycarbonyl, Ethoxycarbonyl, N,N-Dimethyhunino;
oder zwei Reste R⁹ bilden mit dem Kohlenstoffatom, an das sie gebundenen sind, eine Carbonyl- oder Thiocarbonylgruppe,
sowie agrochemisch wirksame Salze davon.

**4.** Verbindungen der Formel (I) gemäß einem oder mehreren der Ansprüche 1 bis 3, **dadurch gekennzeichnet,, dass** die Symbole folgende Bedeutung haben:
R¹ steht für Methyl, Ethyl;
R² steht für Fluor, Chlor, Brom;
R³ steht für Wasserstoff, Fluor, Chlor, Brom, Methyl, Methoxy;
R⁴ bis R⁷ stehen unabhängig voneinander gleichartig oder verschieden für:
Wasserstoff, Fluor, Chlor, Cyano, Methyl;
oder zwei benachbarte Reste R³ bis R⁷ bilden zusammen eine Methylenbis(oxy)- oder Ethan-1,2-diylbis(oxy)-Gruppe, wobei diese Reste einfach oder mehrfach, gleichartig oder verschieden durch Fluor oder Methyl substituiert sein können;
wobei bis zu drei Reste R⁴ bis R⁷ ungleich Wasserstoff sein können und
R⁸ und R⁹ haben die oben angegebenen Bedeutungen
sowie agrochemisch wirksame Salze davon.

**5.** Mittel zur Bekämpfung von unerwünschten Mikroorganismen, **gekennzeichnet durch** einen Gehalt an mindestens einem Arylpyridazin der Formel (I) gemäß einem oder mehreren der Ansprüche 1 bis 4 neben Streckmitteln und/oder oberflächenaktiven Stoffen und/oder Trägerstoffen.

**6.** Mittel gemäß Anspruch 5, **gekennzeichnet durch** einen Gehalt an mindestens einem weiteren agrochemischen Wirkstoff.

**7.** Verfahren zur Bekämpfung von unerwünschten Mikroorganismen, **dadurch** gekonn zeichnet, dass man Verbindungen der Formel (I) gemäß einem oder mehreren der Ansprüche 1 bis 4 bzw, Mittel wie in Anspruch 5 oder 6 definiert auf die unerwünsch ten Mikroorganismen und/oder deren Lebensraum ausbringt,

**8.** Verfahren zur Herstellung von Mitteln zur Bekämpfung von unerwünschten Mikroorga nismen, **dadurch gekennzeichnet, dass** man Verbindungen der Formel (I) gemäß einem oder mehreren der Ansprüche 1 bis 4 mit Streckmitteln und/oder oberflächenaktiven Stoffen vermischt.

**9.** Verwendung von Verbindungen der Formel (I) gemäß einem oder mehreren der Ansprüche 1 bis 4 bzw. Mitteln wie in Anspruch 5 oder 6 definiert zur Bekämpfung von unerwünschten Mikroorganismen.

**10.** Verwendung von Verbindungen der Formel (I) gemäß einem oder mehreren der Ansprüche 1 bis 4 bzw. Mitteln wie in Anspruch 5 oder 6 definiert zur Behandlung von transgenen Pflanzen.

**11.** Verwendung von Verbindungen der Formel (I) gemäß einem oder mehreren der Ansprüche 1 bis 4 zur Behandlung von Saatgut.

**12.** Verwendung von Verbindungen der Formel (I) gemäß einem oder mehreren der Ansprüche 1 bis 4 zur Behandlung von Saatgut transgener Pflanzen,

**13.** Verfahren zum Schutz von Saatgut vor unerwünschten Mikroorganismen durch Verwendung eines, mit wenigstens einer Verbindung der Formel (I), gemäß einem oder mehreren der Ansprüche 1 bis 4, behandelten Saatguts,

**14.** Verfahren zum Herstellen der erfindungsgemäßen Arylpyridazine der Formel (**Ia**) umfassend wenigstens einen der folgenden Schritte (a) bis (d):
a) Umsetzung von Arylessigsäuren der Formel (IIb) mit 2-Haloketonen der Formel (V) gemäß nachfolgendem Reaktionsschema: 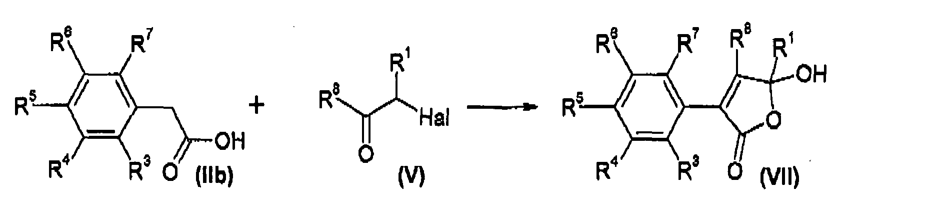
b) Umsetzung von 2-Acyloxyketonen der Formel (VI) gemäß nachfolgendem Reaktionsschema: 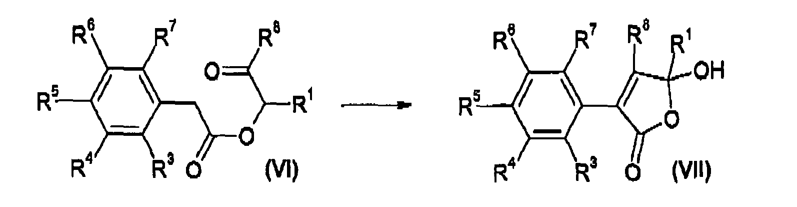
c) Umsetzung von Hydroxylactonen der Formel (VII) mit Hydrazin gemäß nachfolgendem Reaktionsschema; 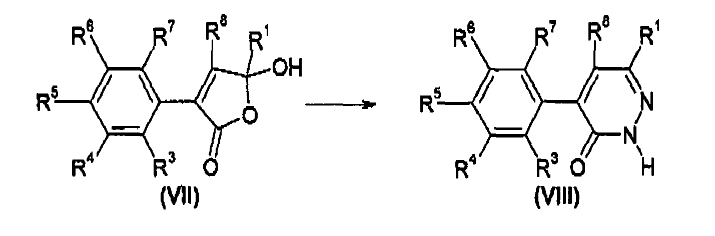
d) Umsetzung von (28)-Pyridazinonen der Formel (VIII) gemäß nachfolgendem Reaktionsschema: 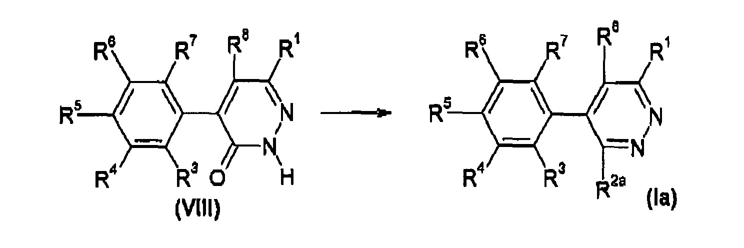
wobei die Reste R¹ und R³ bis R⁹ in den obigen Schemata den oben angegebenen Bedeutungen in einem oder mehreren der Ansprüche 1 bis 4 entsprechen und R^{2a} für Chlor, Brom oder Fluor steht,

**12.** Verbindungen der Formel (VII) 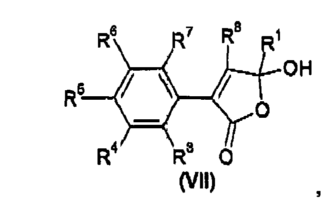 in denen die Resto R¹ bis R⁹ die oben angegebenen Bedeutungen haben.

**13.** Verbindungen der Formel (VIII) 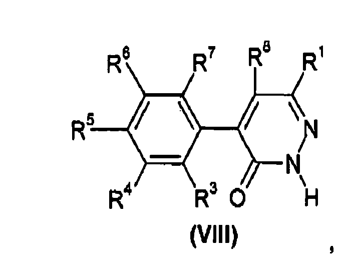 in denen
die Reste R¹ bis R⁹ die oben angegebenen Bedeutungen haben.
